Europäisches Patentamt

European Patent Office

Office européen des brevets

(11) Publication number: **0 042 553**
**B1**

# EUROPEAN PATENT SPECIFICATION

(45) Date of publication of patent specification: **18.09.85**

(21) Application number: **81104513.7**

(22) Date of filing: **11.06.81**

(51) Int. Cl.⁴: **A 61 K 31/455,** A 61 K 31/44, A 61 K 9/00 // (A61K31/455, 31:44, 31:19)

(54) Antitumor compositions.

(30) Priority: **12.06.80 US 156788**

(43) Date of publication of application:
**30.12.81 Bulletin 81/52**

(45) Publication of the grant of the patent:
**18.09.85 Bulletin 85/38**

(84) Designated Contracting States:
**AT BE CH DE FR GB IT LI LU NL SE**

(56) References cited:
JOURNAL OF MEDICINAL CHEMISTRY, vol. 18, November 1975, Washington, DC, USA, B.F. CAIN "Potential antitumor agents. 16. 4'-(acridin-9-ylamino)methanesulfonanilides", pages 1110-1117
JOURNAL OF MEDICINAL CHEMISTRY, vol. 17, September 1974, Washington, DC, USA, B.F. CAIN "Potential antitumor agents. 14. Acridylmethanesulfonanilides", pages 922-930

(73) Proprietor: **Bristol-Myers Company**
**Patent Department 345 Park Avenue**
**New York N.Y. 10022 (US)**

(72) Inventor: **Kaplan, Murray Arthur**
**1026 Glencove Road**
**Syracuse New York (US)**
Inventor: **Granatek, Alphonse Peter**
**8323 Oswego Road**
**Baldwinsville New York (US)**
Inventor: **Shinal, Edward Charles**
**4886 Tanglewood Lane**
**Manlius New York (US)**

(74) Representative: **Kinzebach, Werner, Dr. et al**
**Patentanwälte Reitstötter, Kinzebach und**
**Partner Postfach 780**
**D-8000 München 43 (DE)**

(56) References cited:
CHEMICAL ABSTRACTS, vol. 54, no. 12, June 25, 1960, Column 11648g - 11649c, Columbus, Ohio, USA, M. SAMEJIMA "Solubilizing agents. I. Solubilizing action of acid amides"
CHEMICAL ABSTRACTS, vol. 75, no. 12, September 20, 1971, page 232, abstract 80295m, Columbus, Ohio, USA

**UNLISTED DRUGS, vol. 31, August 1979,**
**Chatham, N.J., USA**
**UNLISTED DRUGS, vol. 27, January 1975,**
**Chatham, N.J., USA**

## Description

Background of the invention

1. Field of the invention

The novel compositions of the present invention possess the advantageous antitumor properties of the known free base compound and in addition have unexpectedly high water-solubility, thus allowing preparation of useful clinical dosage forms for intravenous administration.

2. Description of the prior art

The acridine derivative m-AMSA [4'-9-acridinylamino) methanesulfon-m-anisidide] has been reported by Cain, et al. in *Europ. J. Cancer 10:*539—549 (1974) to possess significant antitumor activity in animal tumor systems. Since then, this compound has been subjected to clinical evaluation with very promising initial results.

When an antitumor agent such as m-AMSA is employed for human clinical use, it is recognized that solubility of the agent is often the controlling factor in determining route of administration and dosage forms. For instance, a water-soluble substance can be generally administered intravenously whereas a water-insoluble material is limited to other forms of parenteral administration such as intramuscular and subcutaneous. A therapeutic agent having water solubility also facilitates preparation of oral and non-intravenous parenteral dosage forms for human administration. Thus, it is decidedly advantageous if a therapeutic agent is water-soluble, particularly when one considers that the most direct route for achieving therapeutic blood levels of a drug within the human body is by intravenous administration.

The free base form of m-AMSA has very limited solubility in water and thus cannot be used as a dosage form for intravenous administration. Attempts have been made to prepare acid addition salts to overcome this solubility problem, but the reported monohydrochloride and monomethanesulfonate salts also proved insufficiently water-soluble for clinical use. The formulation presently in clinical use consists of two sterile liquids combined just prior to use. A solution of m-AMSA in anhydrous N,N-dimethylacetamide is contained in an ampule. A separate vial contains an aqueous L(+)-lactic acid solution for use as a diluent. When mixed the resulting m-AMSA solution is administered by i.v. infusion.

While the present clinical formulation provides an intravenous dosage form, it suffers from several disadvantages. In addition to the obvious difficulties in preparing and administering the dosage form, it contains dimethylacetamide as a vehicle. Dimethylacetamide has been reported to show various toxic symptoms in animals and may thus prove to be unacceptable or undesirable as a pharmaceutical vehicle.

It is accordingly an object of the present invention to provide water-soluble, stable, therapeutically acceptable forms of m-AMSA which can be administered intravenously (as well as by other routes) and which do not contain or require dimethylacetamide as a pharmaceutical vehicle. This object as well as other features and advantages of the invention will be readily apparent to those skilled in the art from the disclosure set forth below.

Summary of the invention

The present invention provides a stable, solid, water-soluble pharmaceutical composition comprising m-AMSA [4'-(9-acridinylamino)-methanesulfon-m-anisidide] in admixture with a pharmaceutically acceptable organic acid and nicotinamide, the m-AMSA and nicotinamide being present in a weight ratio of m-AMSA:nicotinamide from about 1:1 to 1:20, the organic acid and m-AMSA being present in a molar ratio of organic acid:m-AMSA from about 1:1 to 2:1.

These compositions can be reconstituted with water or an aqueous vehicle as stable, aqueous solutions of m-AMSA. More particularly, there are provided (1) mixtures of m-AMSA and a pharmaceutically acceptable organic acid with a solubilizing amount of nicotinamide, (2) mixtures of an organic acid addition salt of m-AMSA with a solubilizing amount of nicotinamide and (3) mixtures of an organic acid addition salt of m-AMSA and a pharmaceutically acceptable organic acid with a solubilizing amount of nicotinamide.

Also provided are processes for preparing the above-mentioned compositions.

Detailed description

Many conventional pharmaceutically acceptable acid addition salts of m-AMSA are only slightly soluble in water and are thus unsuited for preparation of aqueous intravenous solutions. This is evident from literature references to the hydrochloride and methanesulfonate salts as well as from solubility tests carried out by the present inventors on many common organic acid addition salts.

In investigating the solubility properties of m-AMSA organic acid addition salts, we have unexpectedly found that the water solubility of such salts can be markedly increased by combining the salts (or their individual components, i.e. m-AMSA and an organic acid) with nicotinamide. While the degree of solubility enhancement depends on the particular salt of m-AMSA employed, in general the solubility of all organic acid addition salts of m-AMSA is significantly improved by addition of nicotinamide.

The preferred organic acid addition salts of m-AMSA for use in the present invention include the lactate, gluconate, citrate, levulinate, tartrate, pyroglutamate and glucoheptonate salts. Most preferred are the lactate and gluconate m-AMSA salts as these when combined with a solubilizing amount of

0 042 553

nicotinamide may be reconstituted to form high concentration (i.e. ≥75 mg/ml of m-AMSA activity) aqueous solutions suitable for bolus injections.

The compositions provided by the present invention may be employed in the form of either a dry-fill (mixture of dry components) or lyophilized product. Both types of solid dosage form may be conveniently and rapidly reconstituted with water or a sterile aqueous vehicle to provide a true solution of m-AMSA having excellent stability characteristics.

As indicated above, the compositions of the present invention may be considered as belonging to three groups. All three goups, however, involve the same inventive concept, i.e. that nicotinamide significantly enhances the aqueous solubility of m-AMSA organic acid addition salts.

The first type of composition comprises m-AMSA base in admixture with a pharmaceutically acceptable organic acid and nicotinamide. The m-AMSA base and organic acid are in a molar ratio of about 1 to 2 moles of acid per mole of m-AMSA. Sufficient nicotinamide is used so that when the dosage form is reconstituted, a nicotinamide concentration of about 100 to 400, preferably 300 to 400, milligrams per milliliter of aqueous solution will be achieved.

As noted above any pharmaceutically acceptable organic acid may be employed in the present compositions. Examples of suitable acids include citric, lactic, gluconic, levulinic, tartaric, pyroglutamic and glucoheptonic. Most preferred organic acids are lactic and gluconic acids. In addition to use of the acids *per se*, precursors of an organic acid which convert to the desired organic acid in aqueous solution may also be used. Thus, for example, it is convenient to use gluconolactone as a source of gluconic acid since it, unlike gluconic acid, is commercially available in a well-defined crystalline form and rapidly hydrolyzed in water to form gluconic acid. The term "organic acid", therefor, as used herein and in the claims includes such precursors within its scope. Where the organic acids exist in isomeric forms such as resolved and racemic forms, all such forms may be employed in the present compositions.

For preparing unit dosage forms, the m-AMSA is used in any therapeutically useful amount convenient for pharmaceutical administration. A preferred unit dosage form contains about 20 to 100 milligrams of m-AMSA base in admixture with about 1 to 2 molar equivalents of the pharmaceutically acceptable organic acid and 100 to 400, preferably 300—400, milligrams of nicotinamide.

Examples of specific preferred unit dosage forms include the following:

(1) 20—100 mg m-AMSA
1—2 molar equivalents L(+)-lactic acid
100—400 mg nicotinamide;

(2) 20—100 mg m-AMSA
1—2 molar equivalents D(−)-lactic acid
100—400 mg nicotinamide;

(3) 20—100 mg m-AMSA
1—2 molar equivalents DL-lactic acid (or mixture of 50% D(−)-lactic acid and 50% L(+)-lactic acid)
100—400 mg nicotinamide;

(4) 50 mg m-AMSA
26 mg lactic acid
300—400 mg nicotinamide;

(5) 10—30 mg m-AMSA
1—2 molar equivalents citric acid
100—400 mg nicotinamide;

(6) 20—30 mg m-AMSA
1—2 molar equivalents citric acid
300—400 mg nicotinamide;

(7) 20 mg m-AMSA
10 mg citric acid
300—400 mg nicotinamide;

(8) 200—100 mg m-AMSA
1—2 molar equivalents gluconic acid
100—400 mg nicotinamide; and

(9) 75 mg m-AMSA
76 mg gluconic acid
300—400 mg nicotinamide.

4

0 042 553

The second type of composition provided by the present invention comprises an organic acid addition salt of m-AMSA in admixture with nicotinamide, the nicotinamide being present in an amount sufficient to provide on reconstitution with water or aqueous vehicle a nicotinamide concentration of about 100—400 milligrams, preferably 300—400 milligrams, per milliliter of solution.

Solid compositions may be made of any pharmaceutically acceptable organic acid addition salt of m-AMSA since all such salts appear to be significantly more water-soluble when combined with nicotinamide. Examples of particularly suitable m-AMSA salts include the lactate, gluconate, citrate, levulinate, tartrate, pyroglutamate and glucoheptonate salts. Especially preferred are the monolactate and monogluconate salts since compositions of these salts may be reconstituted to form stable, high concentration aqueous solutions suitable for bolus i.v. administration.

The m-AMSA organic acid addition salts may be prepared by methods known in the art. Thus, for example, a mixture of m-AMSA base and a pharmaceutically acceptable organic acid may be dissolved in an inert organic solvent, in a molar ratio of about 1 to 2 moles of acid per mole of m-AMSA base, and the desired salt recovered from solution as by crystallization or precipitation with an antisolvent.

The particular inert organic solvent used to solubilize the m-AMSA base and organic acid is not critical and examples of suitable solvents will be readily apparent to those skilled in the art. Preferred solvents are polar alcohols and ketones such as methanol, ethanol, n-propanol, isopropanol, acetone, n-butanol, 2-butanone, n-pentanol, n-hexanol, diethylene glycol, methyl isobutyl ketone, 3-pentanone, etc. The temperature at which solution is effected is not critical and may range from just above the freezing point to just below the boiling point of the solvent system. Most advantageously, temperatures of around room temperature or above are used. After forming a solution of m-AMSA, it is preferred to carry out a filtration step before recovering the solid salt formed by the addition of the appropriate acid. Standard salt recovery techniques such as crystallization may then be used to obtain the m-AMSA salt. Seed crystals of the desired salt may be added to the reaction mixture to induce and/or enhance crystallization. After recovery, the salt is washed with an inert organic solvent in which it is substantially insoluble and dried by conventional procedures. If desired the salt may be recrystallized from a suitable inert organic solvent to obtain product in a highly purified form.

Preparation of the crystalline monogluconate salt of m-AMSA is disclosed in U.S. Patent application Serial No. 114,809 filed January 24, 1980, now US—A—4322424, and in the section below entitled "Preparation of Starting Materials". U.S. Patent application Serial No. 150,401 filed May 23, 1980, now US—A—4335244, discloses preparation of three crystalline lactate salts which are especially suited for pharmaceutical dosage forms. Preparation of these salts, the crystalline L(+)-monolactate hemiacetonate, the crystalline D(−)-monolactate hemiacetonate and the crystalline DL-monolactate acetone solvate containing about 0.6 to 0.7 moles of acetone per mole of lactate salt, is also described below under "Preparation of Starting Materials". The gluconate salt and three lactate acetone solvates represent the most preferred m-AMSA salts for use in the present compositions.

For preparation of unit dosage forms, the m-AMSA salt is used in an amount sufficient to give a therapeutically useful and pharmaceutically convenient amount of m-AMSA on aqueous reconstitution. A preferred unit dosage form contains 100—400 milligrams, preferably 300—400, of nicotinamide and an amount of m-AMSA salt sufficient to provide from about 20 to 100 milligrams of m-AMSA upon aqueous reconstitution. The precise weight of salt will vary with the particular salt in question and the potency of that salt. As an illustration, if one were using 100% pure m-AMSA monogluconate as the salt, a range of from about 30 to 150 milligrams of gluconate salt would provide 20—100 milligrams of m-AMSA on reconstitution since the m-AMSA content of the gluconate salt is 66.73%. Calculation of a proper weight range of other salts is carried out in a similar manner.

The third type of composition provided by the present invention comprises an organic acid addition salt of m-AMSA in admixture with nicotinamide and a pharmaceutically acceptable organic acid, the nicotinamide being present in an amount sufficient to provide on reconstitution with water or aqueous vehicle a nicotinamide concentration of about 100—400, preferably 300—400, milligrams per milliliter of solution. The maximum solubility appears to be achieved when the m-AMSA salt and organic acid are used in about a 1:1 molar ratio.

Except for the addition of organic acid, this last type of composition is identical to the m-AMSA salt/nicotinamide type described above. Use of the additional amount of organic acid appears to maximize the amount of m-AMSA which can be put into aqueous solution. The same m-AMSA acid addition salts and organic acids may be used as described above for the other two types of m-AMSA compositions. The preferred salts are the gluconate and lactate (particularly the acetone solvates described above) salts and the preferred acids are gluconic acid and lactic acid.

While the pharmaceutically acceptable organic acid which is added to the m-AMSA salt does not have to be the same as the acid used to form the salt, it is preferred that a common organic acid be used.

For preparing unit dosage forms, the m-AMSA salt in the third type of composition is used in an amount sufficient to give a therapeutically useful and pharmaceutically convenient amount of m-AMSA on aqueous reconstitution. A preferred unit dosage form contains an amount of m-AMSA salt sufficient to provide from about 20 to 100 milligrams of m-AMSA upon aqueous reconstitution, about one molar equivalent or organic acid and 100—400, preferably 300—400, milligrams of nicotinamide.

The solid compositions (all three types) provided according to the present invention are reconstituted

5

with sterile water or sterile aqueous vehicle. The preferred unit dosage forms described above may, for example, be reconstituted with one milliliter of sterile water or sterile aqueous vehicle to form relatively concentrated m-AMSA solutions or such solutions may be diluted to form more dilute (e.g. 5 mg/ml m-AMSA activity) solutions. We have found that m-AMSA is incompatible in aqueous solution with chloride, sulfate, phosphate and carbonate ions (insoluble precipitates form). Reconstitution, therefore, should be effected with vehicles substantially free of the above-mentioned ions.

Pharmaceutically acceptable excipients such as mannitol may be optionally employed in the compositions of the present invention to decrease reconstitution time.

Preparation of the solid compositions of the present invention as a dry-fill mixture may be accomplished by simply mixing the desired components. Thus, the m-AMSA/organic acid/nicotinamide composition is prepared by mixing m-AMSA base with a pharmaceutically acceptable organic acid and nicotinamide, the organic acid to m-AMSA molar ratio being from about 1:1 to about 2:1 and the nicotinamide being present in an amount sufficient to provide on aqueous reconstitution a nicotinamide concentration of about 100 to 400 milligrams per milliliter of solution. The m-AMSA salt/nicotinamide composition is prepared by mixing an organic acid addition salt of m-AMSA with nicotinamide, the nicotinamide being present in an amount sufficient to provide on aqueous reconstitution a nicotinamide concentration of about 100 to 400 milligrams per milliliter of solution. Similarly, the m-AMSA salt/organic acid/nicotinamide composition is prepared by mixing an organic acid addition of m-AMSA with nicotinamide and a pharmaceutically acceptable organic acid, the m-AMSA salt and organic acid being preferably in a 1:1 molar ratio and the nicotinamide being present in an amount sufficient to provide on aqueous reconstitution a nicotinamide concentration of about 100—400 milligrams per milliliter of solution. It is of course preferred in preparing dry-fill mixtures to use finely divided, e.g. solid components of 0,25 mm particle size so as to minimize reconstitution time.

For preparation of dry-fill compositions containing gluconic acid as a component, it is preferred to use gluconolactone as a source of gluconic acid in place of gluconic acid *per se*. Similarly, a mixture of 50% D(−)-lactic acid and 50% L(+)-lactic acid is used in place of DL-lactic acid which is a liquid at room temperature.

Preparation of the compositions of the present invention as a lyophilized mixture may be accomplished by forming a solution of the desired components in water and then subjecting such solution, after an optional filtration step to remove solid impurities, to a standard lyophilization process. Lyophilization may be carried out in conventional laboratory or industrial lyophilizers according to methods well-known to those skilled in the art. The reactants and molar ratios used are the same as in the corresponding dry-fill compositions except that liquid organic acids or aqueous solutions of organic acids as well as solid acids may be employed.

The dry-fill and lyophilized compositions provided by the present invention exhibit substantially the same antitumor properties as the prior art m-AMSA forms now being used clinically. Because of their high water-solubility, however, they may be used to prepare single vial clinical dosage forms for intravenous administration which do not contain an undesirable non-aqueous pharmaceutical vehicle such as dimethylacetamide. Furthermore, the present dosage forms allow administration of bolus i.v. injections of m-AMSA which have heretofore not been possible.

The solid compositions of the present invention may be used to prepare oral or non-intravenous parenteral dosage forms as well as the preferred intravenous injectable product. The compositions have excellent stability, both in solid form and in aqueous solution.

In the treatment of mammalian tumors, the compositions may be administered orally or parenterally, but preferably parenterally, in dosages (adjusted for amount of m-AMSA activity) and according to regimens previously disclosed in the literature.

The following examples are given in illustration of, but not in limitation of, the present invention.

Preparation of starting materials

Preparation 1

Sterile crystallization of citric acid

1. Dissolve 20 grams of anhydrous citric acid in 100 ml of acetone.
2. Using aseptic technique, pass the citric acid-acetone solution through a sterile Millipore-Fluoropore® or Mitex® filter. Collect and store the filtrate in a sterile stainless steel container. Wash the filter by passing through it 20 ml of acetone. Add the wash to the filtrate. The combined wash and filtrate is Solution A.
3. Using aseptic technique, pass 2 liters of methylene chloride through a sterile Millipore-Fluoropore®, Celotate® or Mitex® filter. Collect and store the filtrate in a glass or stainless steel container. This is Filtrate B.
4. Place 1.5 liters of the Millipore® filtered methylene chloride (Filtrate B) in a glass or stainless steel container.
5. With rapid stirring add the entire Solution A (see Step 2) over a 5-minute period. Crystals form. Stir an additional 5 minutes.
6. Remove the crystals by sterile, lint-free filtration procedure.

**0 042 553**

7. Wash the crystals with 150 ml of sterile methylene chloride. Avoid contact of the filter cake with excess (moist) air.

8. Vacuum-dry the crystals under sterile conditions at 60—70°C for 24 hours. Expected Yield: 19.0 grams.

9. In a sterile area at a relative humidity of 50 or below, sterilely screen or mill the citric acid to 0.25 mm particle size.

10. Store the 0.25 mm particle size citric acid in a sterile glass container.

Preparation 2

Sterile crystallization of nicotinamide

1. Using aseptic technique, pass 1.5 L of *Skellysolve-B® through a sterile 0.22 micrometer Millipore GS® filter. Collect and store the filtrate in an enclosed, sterile, stainless steel container. Caution: Skellysolve-B® is combustible.

2. Dissolve 10 grams of nicotinamide in 130 ml of absolute ethanol. A solution or near solution is obtained in 10 minutes of stirring.

3. Using aseptic technique, pass the solution through a sterile Fluoropore-FG®, 0.2 micrometer filter. Collect and store the filtrate in a sterile glass or stainless steel container. Cap with a sterile cover.

4. Place one liter of the 0.22 micrometer Millipore® filtered Skellysolve-B® into a stainless steel container.

5. With rapid stirring using a sterile air stirrer, add the Fluoropore-FG®, 0.2 micrometer filtered nicotinamide ethanol solution over a 2—5 minute interval. Crystals form. Stir an additional 5 minutes.
Caution: Mixture is combustible.

6. Remove the crystals by sterile filtration technique. Wash the crystals with 25 ml of the 0.22 micrometer Millipore® filtered Skellysolve-B®.

7. Vacuum dry the crystals in a sterile vacuum oven at 50—50°C for 16—24 hours.

8. Shut off the heat and release the vacuum.

9. Pass the nicotinamide through a sterile 60 mesh stainless steel screen. Yield of screened nicotinamide: 8 grams.

10. Place the screened nicotinamide in a sterile amber bottle. Cover with a sterile screw cap.

* Skellysolve-B® is the tradename ® for a petroleum ether fraction of b.p. 60—80°C consisting essentially of n-hexane and sold by Skelly Oil Co.

Preparation 3

Sterile crystallization of m-AMSA base

1. Dissolve 1.25 grams of m-AMSA base in 10 ml of dimethylacetamide.

2. Using aseptic technique, pass the solution through a suitable, sterile Millipore-Fluoropore® or Mitex® filter. Collect and store the solution in a sterile glass or stainless steel container. Pass 2.5 ml of dimethylacetamide through the filter and add the wash to the filtrate. Mix well. This is Solution A.

3. Using aseptic technique place 200 ml of Sterile Water for Injection, U.S.P. in a sterile glass or stainless steel container. With very rapid stirring add Solution A (Step 2) over a 5 minute period. A thick brown crystalline mass forms. After approximately one half hour of stirring, a change of state is noted and a fluid mass of orange colored microcrystals is obtained.
Vigorously stir an additional two hours.

4. Remove the crystals by lint-free sterile filtration technique. Wash the crystals with 50 ml of Sterile Water for Injection. U.S.P.

5. Vacuum dry the crystals at 55—60°C for 24—48 hours.
Expected Yield: 1.2 grams.

6. Using aseptic technique, pass the crystals through a sterile 0.25 mm stainless steel screen and store the crystals in a sterile screw cap amber bottle.

Properties of m-AMSA base

Karl Fisher Water=0.56%
NMR: No evidence of dimethylacetamide
MP (Cap, uncorr.)=226—230°C; decomposition.

Preparation 4

Crystalline L(+)-Monolactate hemiacetonate of m-AMSA

m-AMSA (400 mg) was dissolved in 35 ml of acetone after 10 minutes of stirring. To this solution there was added with stirring a solution of 450 mg (4 equivalents) of L(+)-lactic acid in 10 ml of acetone. An aliquot of the resulting mixture was glass rod-scratched in a small glass test tube to form crystals. The crystals were added to the reaction mixture and the mixture was stirred for 2 hours at room temperature. The orange crystals which formed were removed by filtration, washed with 10 ml of acetone and vacuum dried at 50°C for 18 hours. Yield of crystalline monolactate: 0.53 grams.

7

Properties of mono L(+)-lactate hemiacetonate

MP: 135—143°C; decomposition.

Spectral analysis: IR, NMR and UV spectra were consistent for a solvated monolactate salt containing 0.5 mole acetone per mole of m-AMSA.

% $H_2O$, KF=0.64

Elemental Analysis: C, 58.44; H, 5.58; N, 7.70; S, 5.95.

Solubility in water: 5 mg/ml.

Stability: 15 mg salt was reconstituted with 10 ml sterile water. The solution was stable for at least 24 hours and showed less that a 6% activity loss after 2 weeks storage at 45°C.

Preparation 5

Sterile crystallization of m-AMSA mono L(+)-lactate hemiacetonate

1. Slurry 1.0 g of m-AMSA free base in 100 ml of acetone at 22—28°C. A solution or near solution is obtained in 10 minutes.

2. Using aseptic technique, pass the acetone solution of m-AMSA through a sterile Millipore-Fluoropore® or Mitex® filter. Collect the filtrate in a sterile glass or stainless steel container. Wash the filter with 15 ml of acetone and add the filtered acetone to the above filtrate. This is Solution A. Use Solution A in Step 5 within 5 hours.

3. Dissolve one gram of L(+)-lactic acid; q.s. to 10 ml. in acetone (100 mg/ml of L(+)-lactic acid). Stir for 5 minutes.

4. Using aseptic technique pass the acetone solution of L(+)-lactic acid through a sterile Millipore-Fluoropore® or Mitex® filter. Collect the filtrate in a sterile glass or stainless steel container. This is Solution B. Do not wash the filter.

5. With moderate stirring add 5.8 ml of Solution B to all of Solution A over a 1—2 minute interval. This represents 2.5 equivalents (0.58 g) of L(+)-lactic acid. Crystals should form in 10 minutes of stirring. If crystals do not form, sterile m-AMSA monolactate hemiacetonate seed crystals may be added or the sides or the container may be scratched with a sterile glass rod to induce crystallization.

6. Stir an additional 1 hour after onset of crystallization.

7. Remove the crystals by lint-free sterile filtration technique. Wash the crystals with 25 ml of acetone previously filtered through a sterile Millipore-Fluoropore® or Mitex® filter.

8. Vacuum-dry the crystals at 50°C for 16—24 hours. Yield of m-AMSA mono L(+)-lactate hemiacetonate salt is 1.1 g.

Preparation 6

Crystalline DL-Monolactate acetone solvate of m-AMSA

m-AMSA base (150 mg) was slurried in 15 ml of acetone for 15 minutes at 45°C. A small amount of insolubles were removed by vacuum filtration through a 15 cm fine glass filter. To the filtrate there was added 0.15 ml of an 80% DL-lactic acid solution with rapid stirring. Crystals formed in about 10 minutes. The mixture was then stirred an additional 30 minutes. The crystals were removed by vacuum filtration through a 15 cm fine glass filter. The crystals were then washed with 2 ml of acetone and vacuum-dried at 50°C for 16 hours. There was obtained 180 mg of the title salt.

Properties

Elemental analysis: C, 59.05%; H, 5.55%; H, 7.85; S, 5.88%.

% $H_2O$ (KF)=1.02.

MP (capillary, uncorrected)=159—166°C; decomposition.

NMR spectrum of the product was consistent for a mono-lactate salt of m-AMSA containing 0.6 mole of acetone per mole of salt. The product contained as an impurity approximately 0.1 mole % of lactyl lactate salt which is formed due to the presence of up to 20% of lactyl lactic acid in ASC purity DL-lactic acid.*

The product salt may be reconstituted with water to give a 5—7.5 mg/ml solution which remains clear at 17°C for at least 6 hours.

Reconstituted aqueous solutions of 5, 7.5 and 10 mg/ml were readily obtained with 3 minutes shaking at 24°C (75°F). Solubility of the salt in water at room temperature is at least 15 mg/ml.

* This may be avoided by using in place of the DL-lactic acid an equimolar mixture of pure L(+)-lactic acid and pure D(−)-lactic acid.

Preparation 7

Sterile crystallization of DL-monolactate acetone solvate of m-AMSA

1. Slurry 1.0 gram of m-AMSA base in 100 ml of acetone at 22—28°C. A solution or near solution is obtained in 10 minutes.

2. Using aseptic technique, pass the acetone solution through a sterile Millipore-Fluoropore® or Mitex® filter.

Collect the filtrate in a sterile glass or stainless steel container.

Wash the filter with 10 ml of acetone and add the filtered acetone to the filtrate. This is Solution A. Use Solution A in step 5 within 5 hours.

3. Dissolve 1 gram of DL-lactic acid (1.18 ml of 85% DL-lactic acid solution); q.s. to 10 ml in acetone (100 mg/ml of DL-lactic acid). Stir for 5 minutes.

4. Using aseptic technique, pass the acetone solution of DL-lactic acid through a sterile Millipore-Fluoropore® or Mitex® filter. Collect the filtrate in a sterile glass or stainless steel container. This is Solution B. Do not wash the filter.

5. With moderate stirring, add 5.8 ml of Solution B to all of Solution A over a 1—2 minute interval. This represents 2.5 equivalents (0.58 g) of DL-lactic acid. Crystals should form in 10 minutes of stirring. If crystals do not form, sterile m-AMSA DL-lactate acetone solvate seed crystals may be added or the sides of the container may be scratched with a sterile glass rod to induce crystallization.

6. Stir an additional 1 hour after onset of crystallization.

7. Remove the crystals by lint-free sterile filtration technique. Wash the crystals with 10 ml of acetone previously filtered through a sterile Millipore-Fluoropore® or Mitex® filter.

8. Vacuum dry the crystals at 50°C for 16—24 hours. Expected yield of DL-lactate acetone solvate is 1.1 grams.

Properties
IR and NMR consistent for monolactate m-AMSA salt having ∞0.7 mole acetone solvated and approximately 0.1 mole % lactyl lactate as impurity.
MP=159—166°C; decomposition.
% m-AMSA in salt=72% (based on HPLC assay).
Solubility in water=25 mg/ml at room temperature.


Preparation 8
Sterile crystallization of m-AMSA D(−)-monolactate hemiacetonate

1. Slurry 1.0 gram of m-AMSA base in 100 ml of acetone at 22—28°C. A solution or near-solution is obtained in 10 minutes.

2. Using aseptic technique, pass the acetone solution of m-AMSA through a sterile Millipore-Fluoropore® or Mitex® filter. Collect the filtrate in a sterile glass or stainless steel container. Wash the filter with 10 ml of acetone and add the Millipore®-filtered acetone to the filtrate. This is Solution A. Use Solution A in step 5 within 5 hours.

3. Dissolve 1 gram of D(−)-lactic acid in sufficient acetone to provide 10 ml of acetone solution (100 mg/ml of D(−)-lactic acid). Stir for 5 minutes.

4. Using aseptic technique, pass the acetone solution of D(−)-lactic acid through a Millipore-Fluoropore® or Mitex® filter. Collect the filtrate in a sterile glass or stainless steel container. This is Solution B. Do not wash the filter.

5. With moderate stirring add 5.8 ml of solution B to all of Solution A over a 1—2 minute interval. This represents 2.5 equivalents (0.58 g) of D(−)-lactic acid. Crystals should form in 10 minutes of stirring. If crystals do not form, sterile m-AMSA D(−)-lactate acetone solvate seed crystals may be added or the sides of the container may be scratched with a sterile glass rod to induce crystallization.

6. Stir an additional 1 hour after onset of crystallization.

7. Remove the crystals by suitable lint-free sterile filtration technique. Wash the crystals with 10 ml of acetone previously filtered through a sterile Millipore-Fluoropore® or Mitex® filter.

8. High vacuum dry the crystals at 50°C for 16—24 hours. Usual yield of m-AMSA D(−)-monolactate acetone solvate is 1.1 grams.

Properties
IR and NMR consistent for monolactate m-AMSA salt having ∞0.4 mole acetone solvated to salt.
MP (capillary, uncorrected): 180—184°C; decomposition.
% m-AMSA in salt: 78.4% (based on HPLC assay).
Elemental analysis: C, 59.47; H, 5.20; N, 8.41; S, 6.46.
% $H_2O$(KF)=0.39.


Preparation 9
Crystalline m-AMSA monogluconate salt
Delta gluconolactone (0.89 g; 0.005 mole) was dissolved in 0.5 ml of water. m-AMSA base (1.95 g; 0.005 mole) and 100 ml of ethanol were added, and the mixture was then refluxed for a short time, i.e. about 5—10 minutes. The resulting solution was allowed to stand overnight whereupon crystalline material separated from solution. The product was recrystallized from 100 ml of ethanol to give 1.10 g of crystalline m-AMSA monogluconate salt.

Properties
m-AMSA content by U.V.=62.6% (theoretical content is 66.6%).
gluconic acid content by U.V.=36.9
gluconolactone content by U.V.=1.1
Solubility in water: 30 mg/ml at 50—60°C; 25 mg/ml at room temperature.

9

# 0 042 553

When dissolved in water at a concentration of 7.1 µg/ml, the gluconate salt exhibits ultraviolet absorption peaks at 208 nm (O.D.=0.527), 247.5 nm (O.D.=0.567), 263 nm (O.D.=0.425) and 412 nm (O.D.=0.121).

The IR spectrum was consistent for the monogluconate salt of m-AMSA.

Preparation 10

Sterile crystallization of mannitol

1.	Place 90 ml. of sterile water into a glass container. Start stirring and maintain the temperature in the range of 20—26°C. Add and dissolve 13.6 grams of mannitol. Maintain stirring until solution is obtained.
2.	Continue stirring and add 1.4 grams of Darko KB® (activated carbon). Maintain stirring for 1 hour.
3.	Remove the carbon by filtration. Wash the carbon cake with 5 ml of sterile water. Add the wash to the filtrate.
4.	Using nitrogen pressure and aseptic technique, pass the filtrate through a sterile, pyrogen-free 0.22 micrometer Millipore® filter into a sterile, pyrogen-free glass container.
5.	Using aseptic technique, add to the filtrate, with rapid stirring and over a 10 minute interval, 500 ml of acetone which has been filtered through a sterile 0.22 micron Millipore® filter. Crystals form. (Alternatively, the filtrate may be added over a 10 minute interval to 500 ml of rapidly stirring acetone which has been filtered through a sterile 0.22 micrometer Millipore® filter). Maintain stirring for 0.5 hour at room temperature.
6.	Remove the crystals by suitable filtration using aseptic technique.
7.	Wash the crystals on the filter with two 20 ml portions of acetone which has been filtered through a sterile 0.22 micrometer Millipore® filter.
8.	Using aseptic technique remove the crystals from the filter and air dry at 100—115°C for 24 hours. Yield: Approximately 11.4 grams (84% yield).
9.	Store the crystals in a sterile, pyrogen-free amber glass container capped with a metal screw cap containing a Teflon® liner.

Example 1

Lyophilized mixture of m-AMSA L(+)-monolactate hemiacetonate salt and nicotinamide (20 mg. m-AMSA activity vials; 100 vials)

### Formula

| Ingredient | Per vial |
|---|---|
| m-AMSA L(+)-monolactate hemiacetonate | *0.02 g m-AMSA activity |
| nicotinamide | 0.20 g |

Procedure

1.	Place 65 ml sterile water in a glass container.
2.	With moderate stirring, add 20 grams of nicotinamide. A solution is obtained.
3.	Add, with stirring, the equivalent of 2 grams of m-AMSA activity as m-AMSA L(+)-monolactate hemiacetonate. A solution is obtained. Stir for 5 minutes.
4.	Add sterile water to qs. to 100 ml. Stir for 10 minutes.
5.	Using aseptic technique pass the solution through a sterile 0.22 micrometer Millipore® filter. Collect the filtrate in a sterile amber screw cap bottle. The solution may be kept for 24 hours at room temperature if required.
6.	Fill sterile vials with 2 ml of the solution. An overfill may be required to meet needle-vial-syringe holdup volumes.
7.	Using aseptic technique, lyophilize the vials in a suitable sterile lyophilizer such as a Repp® Sublimator (shelf temp. 24—27°C (75—80°F). After completion of the lyophilization, maintain the vacuum and a shelf temp. of 40—50°C for 18—24 hours.
8.	Turn off the heat and break the vacuum with sterile air.
9.	Stopper the vials with sterile rubber enclosures and cap with aluminum seals.
10.	The vials may be reconstituted with an amount of sterile water to yield 20—40 mg/ml m-AMSA activity as the L(+)-monolactate. If required, the reconstituted solution may be kept in the dark at ambient room temperature for 24 hours.

* The amount of m-AMSA L(+)-monolactate hemiacetonate needed is a function of the potency of the salt, overfill required and needle-syringe-vial holdup. For example, the m-AMSA content of the hemiacetonate salt is 76.31%. Thus, to provide 0.02 grams of m-AMSA activity, one would need 0.026 g of 100% pure hemiacetonate salt. This amount is then adjusted for the actual potency of salt, overfill required, etc.

Caution: Solutions of m-AMSA are incompatible with chloride, sulfate, phosphate and carbonate ions. Precipitates form.

10

Example 2
Dry-fill mixture of m-AMSA L(+)-monolactate hemiacetonate and nicotinamide (20 mg m-AMSA activity vials; 100 vials)

Formula

| Ingredient | Per vial |
|---|---|
| Sterile m-AMSA L(+)-monolactate hemiacetonate, 0,25 mm (60 Mesh) | *0.020 g activity |
| Sterile nicotinamide, 0,25 mm (60 Mesh) | 0.20 g |

Procedure
1. Using aseptic technique, blend the sterile 0,25 mm (60 mesh) particle size nicotinamide and m-AMSA lactate hemiacetonate in a sterile blender. Mix for 2 hours.
2. If lumps are present, pass the blend through a sterile mill (0,25 mm (60 mesh)).
3. Collect and store the blend in a sterile amber screw-cap bottle.
4. Obtain the required assays.
5. Using aseptic technique, fill the required amount of the sterile blend into sterile vials. Cap with sterile enclosures and seal with aluminum seals.
6. The amount of sterile water required for reconstitution is that amount which will give 20—40 mg/ml of m-AMSA activity.

* See footnote of Example 1.

Example 3
Dry-fill mixture of m-AMSA, citric acid and nicotinamide (20 mg m-AMSA activity vials)

Formula

| Ingredient | Per vial |
|---|---|
| Sterile m-AMSA base, 0,25 mm (60 mesh) | *0.02 g |
| Sterile citric acid, 0,25 mm (60 mesh) | 0.01 g |
| Sterile nicotinamide, 0,25 mm (60 mesh) | 0.20 g |

Procedure
1. Using aseptic technique, place the required amounts of sterile 0,25 mm particle size (60 mesh) m-AMSA base, citric acid and nicotinamide in a sterile blender.
2. Blend for 2 hours.
3. Drop the blend into a sterile amber screw-cap glass bottle.
4. Obtain required assays.
5. Using aseptic technique, fill the required amount of sterile blend into sterile vials. Cap with sterile rubber enclosures and seal with aluminum seals.
6. The amount of sterile water required for reconstitution is that amount which will give a 20 mg/ml solution of m-AMSA activity as the monocitrate salt. The solution may be stored in the dark for 24 hours at room temperature.

* Actual amount of m-AMSA base used will depend on potency.

Example 4
Lyophilized mixture of m-AMSA, citric acid and nicotinamide (20 mg m-AMSA activity vials; 100 vials)

Formula

| Ingredient | Per vial |
|---|---|
| m-AMSA base | *0.02 g |
| citric acid, anhydrous | 0.001 g |
| nicotinamide | 0.20 g |

Procedure
1. Place 70 ml sterile water in a clean glass or stainless steel container.
2. With moderate stirring, add and dissolve the nicotinamide (20 g) and the anhydrous citric acid (1 g; or the equivalent as 1.086 g citric acid monohydrate). Stir for 5 minutes.

* Actual amount used dependent on potency.

11

# 0 042 553

3. With rapid stirring, add the m-AMSA base (2 g) over a 5 minute interval. A solution or near solution is obtained. Stir an additional 5 minutes.

4. Stop stirring and add sterile water to qs. to 100 ml. Stir for 15 minutes. A solution or near solution is obtained.

5. Using aseptic technique, pass the solution of m-AMSA monocitrate through a sterile 0.22 micrometer Millipore® filter. Collect and store the filtrate in a sterile screw-cap amber bottle. If required, the solution may be stored in the dark at room temperature for up to 24 hours.

6. Fill 2 ml (20 mg of m-AMSA activity) of the solution into sterile vials.

7. Using aseptic technique, lyophilize the vials in a suitable lyophilizer such as a Repp® Sublimator (shelf temperature 24—27°C (75—80°F). At the completion of the lyophilization cycle, raise the shelf temperature to 40—50°C and maintain the vacuum for 16—24 hours.

8. Shut off the heat and release the vacuum with sterile air.

9. Cap the vials with sterile rubber enclosures. Seal with aluminum seals.

10. The vials may be reconstituted with an amount of sterile water to give 20 mg/ml of m-AMSA activity as the m-AMSA monocitrate salt. If required, the solution may be stored in the dark at room temperature for up to 24 hours.

Example 5

Dry-fill mixture of m-AMSA, nicotinamide and L(+)-lactic acid (50 mg m-AMSA activity vials)

### Formula

| Ingredient | Per vial |
|---|---|
| Sterile m-AMSA base, 0,25 mm (60 mesh) | 50 mg |
| Sterile nicotinamide, 0,25 mm (60 mesh) | 300 mg |
| Sterile L(+)-lactic acid, 0,25 mm (60 mesh) | 26 mg |

Procedure

Using aseptic technique blend sterile m-AMSA, nicotinamide and L(+)-lactic acid and fill blend into sterile vials. Cap and seal. For reconstitution, qs. to 1 ml with sterile water to give a 50 mg/ml m-AMSA activity solution. This solution can be used for bolus injection or diluted with sterile water to give 1, 2, 5 or 10 mg/ml m-AMSA activity solutions.

The above mixture can be rapidly (<3 min.) reconstituted with sterile water. The 50 mg/ml solution and 1, 2, 5 and 10 mg/ml dilutions remain clear for at least 24 hours at 15—20°C.

Example 6

Dry-fill mixture of m-AMSA, nicotinamide and L(+)-lactic acid

### Formula

| Ingredient | Per vial |
|---|---|
| Sterile m-AMSA, 0,25 mm (60 mesh) | 50 mg |
| Sterile nitocinamide, 0,25 mm (60 mesh) | 350 mg |
| Sterile L(+)-lactic acid, 0,25 mm (60 mesh) | 13 mg |

Procedure

The above mixture is made according to the general procedure of Example 5. With only 1 mole L(+)-lactic acid per mole m-AMSA, reconstitution time is somewhat greater and the 5 and 10 mg/ml dilutions do not remain clear for 24 hours at 15—20°C.

Example 7

Dry-fill mixture of m-AMSA mono L(+)-lactate hemiacetonate, nicotinamide and L(+)-lactic acid (50 mg m-AMSA activity vials)

### Formula

| Ingredient | Per vial |
|---|---|
| Sterile m-AMSA L(+)-monolactate hemiacetonate, 0,25 mm (60 mesh) | *50 mg m-AMSA activity |
| Sterile nicotinamide, 0,25 mm (60 mesh) | 400 mg nicotinamide |
| Sterile L(+)-lactic acid, 0,25 mm (60 mesh) | 13 mg |

Procedure

The above mixture is made according to the general procedure of Example 5.

\* Amount of salt actually used is dependent on potency. For 100% pure salt, calculate wgt. needed by formula

$$\frac{\text{mg m-AMSA activity desired}}{.7631}$$

Example 8

Dry-fill mixture of m-AMSA DL-monolactate acetone solvate and nicotinamide

### Formula

| Ingredient | Per vial |
|---|---|
| Sterile m-AMSA DL-monolactate acetone solvate containing 0.6—0.7 moles acetone per mole of lactate salt, 0,25 mm (60 mesh) | 0.075 g of m-AMSA activity |
| Sterile nicotinamide, 0,25 mm (60 mesh) | 0.30 g |

Procedure

1. Using aseptic technique blend the sterile 60 mesh nicotinamide and m-AMSA salt in a sterile blender. Mix for 2 hours.
2. If lumps are present, pass the blend through a sterile 60 mesh stainless steel screen or a sterile mill (0,25 mm (60 mesh)).
3. Collect and store the blend in a sterile amber screw-cap bottle.
4. Obtain the required assays.
5. Using aseptic technique, fill the required amount of the sterile blend into sterile vials. Cap with sterile enclosures and seal with aluminum seals.
6. For administration, the vial is reconstituted with sterile water, qs. to 1 ml, to yield 75 mg/ml of m-AMSA activity. This in turn may be diluted with sterile water or sterile 5% dextrose solution to yield 7.5 mg/ml or less of m-AMSA. These solutions may be stored at ambient room temperature and should be used within 24 hours.

Example 9

Using the general procedures of Examples 1—9, the following compositions may be prepared:

9A Dry-fill mixture

### Formula

| Ingredient | Per vial |
|---|---|
| m-AMSA base | 75 mg |
| nicotinamide | 350 mg |
| L(+)-lactic acid | 39 mg |

9B Dry-fill mixture

### Formula

| Ingredient | Per vial |
|---|---|
| m-AMSA base | 20 mg |
| nicotinamide | 100 mg |
| L(+)-lactic acid | 10.4 mg |

9C Lyophilized mixture

### Formula

| Ingredient | Per vial |
|---|---|
| m-AMSA L(+)-monolactate hemiacetonate | *50 mg m-AMSA activity |
| nicotinamide | 350 mg |
| D(−)-lactic acid | 13 mg |

9D Lyophilized mixture

\* See footnote, Example 7.

Formula

| Ingredient | Per vial |
|---|---|
| m-AMSA D(−)-monolactate hemiacetonate | *50 mg m-AMSA activity |
| nicotinamide | 350 mg |
| D(−)-lactic acid | 13 mg |

* See footnote, Example 7.

9E  Lyophilized Mixture

Formula

| Ingredient | Per vial |
|---|---|
| m-AMSA DL-monolactate acetone solvate | 50 mg m-AMSA activity |
| nicotinamide | 350 mg |
| DL-lactic acid | 13 mg |

9F  Dry-fill mixture

Formula

| Ingredient | Per vial |
|---|---|
| m-AMSA gluconate | 75 mg m-AMSA activity |
| nicotinamide | 360 mg |
| gluconic acid | 34 mg |

9G  Lyophilized mixture

Formula

| Ingredient | Per vial |
|---|---|
| m-AMSA gluconate | 75 mg m-AMSA activity |
| nicotinamide | 360 mg |
| gluconic acid | 38 mg |

9H  Lyophilized mixture

Formula

| Ingredient | Per vial |
|---|---|
| m-AMSA | 75 mg m-AMSA activity |
| nicotinamide | 350 mg |
| gluconolactone | 65 mg |

9I  Lyophilized mixture

Formula

| Ingredient | Per vial |
|---|---|
| m-AMSA | 50 mg m-AMSA activity |
| L-pyroglutamic acid | 17—35 mg |
| nicotinamide | 350 mg |

9J  Lyophilized mixture

Formula

| Ingredient | Per vial |
|---|---|
| m-AMSA | 50 mg m-AMSA activity |
| tartaric acid | 2 molar equivalents |
| nicotinamide | 300 mg |

9K  Dry-fill mixture

14

Formula

| Ingredient | Per vial |
|---|---|
| m-AMSA | 50 mg m-AMSA activity |
| glucoheptonic acid | 2 molar equivalents |
| nicotinamide | 350 mg |

9L   Lyophilized mixture

Formula

| Ingredient | Per vial |
|---|---|
| m-AMSA | 50 mg m-AMSA activity |
| levulinic acid | 2 molar equivalents |
| nicotinamide | 300 mg |

9M   Lyophilized mixture

Formula

| Ingredient | Per vial |
|---|---|
| m-AMSA gluconate | 50 mg m-AMSA activity |
| nicotinamide | 300 mg |

9N   Lyophilized mixture

Formula

| Ingredient | Per vial |
|---|---|
| m-AMSA DL-monolactate acetone solvate | 75 mg m-AMSA activity |
| nicotinamide | 350 mg |

9O   Dry-fill mixture

Formula

| Ingredient | Per vial |
|---|---|
| m-AMSA D(−)-monolactate hemiacetonate | 75 mg m-AMSA activity |
| nicotinamide | 350 mg |

9P   Dry-fill mixture

Formula

| Ingredient | Per vial |
|---|---|
| m-AMSA L(+)-monolactate hemiacetonate | 50 mg m-AMSA activity |
| nicotinamide | 300 mg |

**Claims for the Contracting States: BE, CH, DE, FR, GB, IT, LI, LU, NL, SE**

1. A stable, solid, water-soluble pharmaceutical composition comprising m-AMSA [4'-(9-acridinylamino)-methanesulfon-m-anisidide] in admixture with a pharmaceutically acceptable organic acid and nicotinamide, the m-AMSA and nicotinamide being present in a weight ratio of m-AMSA:nicotinamide from about 1:1 to 1:20, the organic acid and m-AMSA being present in a molar ratio of organic acid:m-AMSA from about 1:1 to 2:1.

2. A composition as claimed in claim 1 wherein the organic acid is tartaric acid, lactic acid, levulinic acid, citric acid, gluconic acid, pyroglutamic acid or glucoheptonic acid.

3. A composition according to claim 2, wherein the organic acid is pyroglutamic acid.

4. A composition as claimed in claim 2 wherein the organic acid is lactic acid.

5. A composition as claimed in claim 2 wherein the organic acid is gluconic acid.

6. A stable, solid, water-soluble pharmaceutical composition comprising an organic acid addition salt of m-AMSA with a pharmaceutically acceptable organic acid, in admixture with nicotinamide, the m-AMSA and nicotinamide being present in a ratio of about 2:40 to 1:1.

7. A composition as claimed in claim 6 wherein the m-AMSA salt is m-AMSA lactate, m-AMSA gluconate, m-AMSA citrate, m-AMSA levulinate, m-AMSA tartrate, m-AMSA pyroglutamate or m-AMSA glucoheptonate.

8. A composition as claimed in claim 6 wherein the m-AMSA salt is m-AMSA lactate.

9. A composition as claimed in Claim 8 wherein the m-AMSA lactate salt is m-AMSA L(+)-monolactate hemiacetonate.

10. A composition as claimed in claim 8 wherein the m-AMSA lactate salt is m-AMSA D(−)-monolactate hemicacetonate.

11. A composition as claimed in claim 8 wherein the m-AMSA lactate is m-AMSA DL-monolactate acetonate having about 0.6—0.7 moles of acetone per mole of lactate.

12. A composition as claimed in claim 6 wherein the m-AMSA salt is m-AMSA gluconate.

13. A stable, solid, water-soluble pharmaceutical composition comprising an organic acid addition salt of m-AMSA with a pharmaceutically acceptable organic acid, in admixture with nicotinamide and a pharmaceutically acceptable organic acid, the m-AMSA and nicotinamide being present in a ratio of about 2:40 to 1:1.

14. A composition as claimed in claim 13 wherein the m-AMSA salt and organic acid are in a molar ratio of about 1:1.

15. A composition as claimed in claim 13 or claim 14 wherein the m-AMSA salt is m-AMSA lactate, m-AMSA gluconate, m-AMSA citrate, m-AMSA levulinate, m-AMSA tartrate, m-AMSA pyroglutamate or m-AMSA glucoheptonate and the organic acid is tartaric acid, lactic acid, levulinic acid, citric acid, gluconic acid, pyroglutamic acid or glucoheptonic acid.

16. A composition as claimed in claim 13 or claim 14 wherein the m-AMSA salt is m-AMSA lactate and the organic acid is lactic acid.

17. A composition as claimed in claim 16 wherein the m-AMSA lactate is m-AMSA L(+)-monolactate hemiacetonate.

18. A composition as claimed in claim 16 wherein the m-AMSA lactate is m-AMSA DL-monolactate acetonate having about 0.6—0.7 moles of acetone per mole of lactate.

19. A composition as claimed in claim 16 wherein the m-AMSA lactate is m-AMSA D(−)-monolactate hemiacetonate.

20. A composition as claimed in claim 13 or claim 14 wherein the m-AMSA salt is m-AMSA gluconate and the organic acid is gluconic acid.

21. A pharmaceutical composition as claimed in claim 4 in unit dosage form which comprises about 20 to 100 milligrams of m-AMSA in admixture with about 1 to 2 molar equivalents of lactic acid and 100 to 400 milligrams of nicotinamide.

22. A composition according to claim 21 wherein the lactic acid is L(+)-lactic acid.

23. A composition according to claim 21 wherein the lactic acid is DL-lactic acid.

24. A composition according to claim 21 wherein the lactic acid is D(−)-lactic acid.

25. A composition according to claim 21 comprising about 20 to 100 milligrams m-AMSA, 100—400 milligrams of nicotinamide and 13—26 milligrams of lactic acid.

26. A composition according to claim 21 comprising about 50 milligrams m-AMSA, 300—400 milligrams nicotinamide and 26 milligrams lactic acid.

27. A pharmaceutical composition as claimed in claim 2 in unit dosage form which comprises about 10 to 30 milligrams m-AMSA in admixture with about 1 to 2 molar equivalents of citric acid and 100 to 400 milligrams of nicotinamide.

28. A composition according to claim 27 comprising about 20 to 30 milligrams m-AMSA, 1 to 2 molar equivalents of citric acid and 300 to 400 milligrams of nicotinamide.

29. A composition according to claim 27 comprising about 20 milligrams m-AMSA, 10 mg citric acid and 300 to 400 milligrams of nicotinamide.

30. A pharmaceutical composition as claimed in claim 2 in unit dosage form which comprises about 20 to 100 milligrams of m-AMSA in admixture with about 1 to 2 molar equivalents of gluconic acid or gluconolactone and 100 to 400 milligrams of nicotinamide.

31. A composition according to claim 30 comprising about 75 milligrams m-AMSA, 37—74 milligrams of gluconic acid or gluconolactone and 300—400 milligrams of nicotinamide.

32. A pharmaceutical composition as claimed in any one of claims 6—12 in unit dosage form which comprises about 100 to 400 milligrams of nicotinamide in admixture with an amount of an organic acid addition salt of m-AMSA sufficient to provide upon water reconstitution about 20 to 100 milligrams of m-AMSA.

33. A pharmaceutical composition as claimed in anyone of claims 13 to 20 in unit dosage form comprising an amount of an organic acid addition salt of m-AMSA sufficient to provide upon water reconstitution about 20 to 100 milligrams of m-AMSA in admixture with about one molar equivalent of a pharmaceutically acceptable organic acid and 100 to 400 milligrams of nicotinamide.

34. A composition according to claim 33 comprising an amount of m-AMSA lactate sufficient to provide upon water reconstitution about 50 milligrams of m-AMSA, one molar equivalent of lactic acid and 300 to 400 milligrams of nicotinamide.

35. A pharmaceutical composition as claimed in claim 2 in unit dosage form which comprises 50 mg m-AMSA, 17—35 mg L-pyroglutamic acid and 350 mg nicotinamide.

36. A process for preparing a composition according to anyone of claims 1—5, 21—31 and 35 which comprises

16

A) mixing m-AMSA with a pharmaceutically acceptable organic acid and nicotinamide in the desired ratios, or
B) the steps of
a) forming an aqueous solution of an organic acid addition salt of m-AMSA, a pharmaceutically acceptable organic acid and nicotinamide in the desired ratios; and
b) lyophilizing the so-produced aqueous solution.

37. The process according to claim 36 wherein the organic acid in process A is D(−)-lactic acid, L(+)-lactic acid, a mixture of 50 % D(−)- and 50 % L(+)-lactic acid or gluconolactone.

38. The process according to claim 36 wherein the aqueous solution of step a) is filtered prior to lyophilization.

39. The process according to claim 36 or claim 38 wherein the organic acid in step a) is lactic acid, gluconic acid or gluconolactone.

40. A process for preparing a composition according to anyone of claims 6 to 12 and 32 which comprises

A) mixing an organic acid addition salt of m-AMSA with nicotinamide in the desired ratio; or
B) the steps of
a) forming an aqueous solution of an organic acid addition salt of m-AMSA and nicotinamide in the desired ratio; and
b) lyophilizing the so-produced solution.

41. The process according to claim 40 wherein the aqueous solution of step a) is filtered prior to lyophilization.

42. A process for preparing a composition according to anyone of claims 13—20, 33 and 34, which comprises

A) mixing an organic acid salt of m-AMSA with nicotinamide and a pharmaceutically acceptable organic acid in the desired ratios; an amount sufficient to provide on reconstitution with water a nicotinamide concentration of or
B) the steps of
a) forming an aqueous solution of an organic acid addition salt of m-AMSA, nicotinamide and a pharmaceutically acceptable organic acid in the desired ratios and
b) lyophilizing the so-produced aqueous solution.

43. The process according to claim 42 wherein the m-AMSA salt is m-AMSA lactate and the organic acid is D(−)-lactic acid, L(+)-lactic acid or a mixture of 50 % D(−)- and 50 % L(+)-lactic acid.

44. The process according to claims 42 or 43 wherein the aqueous solution of step a) is filtered prior to lyophilization.

**Claims for the Contracting State AT**

1. Process for preparing a stable, solid, water-soluble pharmaceutical composition of m - AMSA[4' - (9 - acridinylamino) - methanesulfon - m - anisidide] in admixture with a pharmaceutically acceptable organic acid and nicotinamide, the m-AMSA and nicotimamide being present in a weight ratio of m-AMSA: nicotinamide from about 1:1 to 1:20, the organic acid and m-AMSA being present in a molar ratio of organic acid: m-AMSA from about 1:1 to 2:1, which comprises

A) mixing m-AMSA with a pharmaceutically acceptable organic acid and nicotinamide in the desired ratios or
B) the steps of
a) forming an aqueous solution of an organic acid addition salt of m-AMSA, a pharmaceutically acceptable organic acid and nicotinamide in the desired ratios; and
b) lyophilizing the so-produced aqueous solution.

2. Process according to claim 1 wherein the organic acid used is tartaric acid, lactic acid, levulinic acid, citric acid, gluconic acid, pyroglutamic acid or glucoheptonic acid.

3. A process according to claim 2, wherein the organic acid is pyroglutamic acid.

4. A process as claimed in claim 2, wherein the organic acid is lactic acid.

5. A process as claimed in claim 2, wherein the organic acid is gluconic acid.

6. A process as claimed in claim 4 for preparing a pharmaceutical composition in unit dosage form which comprises about 20 to 100 milligrams of m-AMSA in admixture with about 1 to 2 molar equivalents of lactic acid and 100 to 400 milligrams of Nicotinamide.

7. A process according to claim 6, wherein the lactic acid is L(+)-lactic acid.

8. A process according to claim 6, wherein the lactic acid is DL-lactic acid.

9. A process according to claim 6, wherein the lactic acid is D(−)-lactic acid.

10. A process according to claim 6, for preparing a pharmaceutical composition which comprises about 20 to 100 milligrams m-AMSA, 100—400 milligrams of nicotinamide and 13—26 milligrams of lactic acid.

11. A process according to claim 6 for preparing a pharmaceutical composition which comprises about 50 milligrams m-AMSA, 300—400 milligrams nicotinamide and 26 milligrams lactic acid.

12. A process according to claim 2 for preparing a pharmaceutical composition in unit dosage form which comprises about 10 to 30 milligrams of m-AMSA in admixture with about 1 to 2 molar equivalents of citric acid and 100 to 400 milligrams of nicotinamide.

13. A process according to claim 12 for preparing a pharmaceutical composition which comprises about 20 to 30 milligrams m-AMSA, 1 to 2 molar equivalents of citric acid and 300 to 400 milligrams of nicotinamide.

14. A process according to claim 12 for preparing a pharmaceutical composition which comprises about 20 to 100 milligrams m-AMSA, 10 mg citric acid and 300—400 milligrams of nicotinamide.

15. A process according to claim 2 for preparing a pharmaceutical composition in unit dosage form which comprises about 20 to 100 milligrams of m-AMSA in admixture with about 1 to 2 molar equivalents of gluconic acid or gluconolactone and 100 to 400 milligrams of nicotinamide.

16. A process according to claim 15 for preparing a pharmaceutical composition which comprises about 75 milligrams m-AMSA, 37—74 milligrams of gluconic acid or gluconolactone and 300—400 milligrams of nicotinamide.

17. A process as claimed in claim 2 for preparing a pharmaceutical composition in unit dosage form which comprises 50 mg m-AMSA, 17—35 mg L-pyroglutamic acid and 350 mg nicotinamide.

18. A process according to anyone of the preceding claims wherein the organic acid in process A is D(−)-lactic acid, L(+)-lactic acid, a mixture of 50% D-(−)- and 50% L(+)-lactic acid or gluconolactone.

19. A process according to anyone of the preceding claims wherein the aqueous solution of step a) is filtered prior to lyophilization.

20. A process according to claims 1—17 or claim 19 wherein the organic acid in step a) is lactic acid, gluconic acid or gluconolactone.

21. A process for preparing a stable, solid, water-soluble pharmaceutical composition comprising an organic acid addition salt of m-AMSA with a pharmaceutically acceptable organic acid, in admixture with nicotinamide, the m-AMSA and nicotinamide being present in a ratio of about 2:40 to 1:1, which comprises

    A) mixing an organic acid addition salt of m-AMSA with nicotinamide in the desired ratio or
    B) the steps of
        a) forming an aqueous solution of an organic acid addition salt of m-AMSA and nicotinamide in the desired ratio; and
        b) lyophilizing the so-produced solution.

22. A process according to claim 21, wherein the m-AMSA salt is m-AMSA lactate, m-AMSA gluconate, m-AMSA citrate, m-AMSA levulinate, m-AMSA tartrate, m-AMSA pyroglutamate or m-AMSA glucoheptonate.

23. A process according to claim 21, wherein the m-AMSA salt is m-AMSA lactate.

24. A process according to claim 23, wherein the m-AMSA lactate salt is m-AMSA L(+)-monolactate hemiacetonate.

25. A process according to claim 23, wherein the m-AMSA lactate salt is m-AMSA D(−)-monolactate hemiacetonate.

26. A process according to claim 23, wherein the m-AMSA lactate is m-AMSA DL-monolactate acetonate having about 0.6—0.7 moles of acetone per mole of lactate.

27. A process according to claim 21, wherein the m-AMSA salt is m-AMSA gluconate.

28. A process according to anyone of claims 21—27 for preparing a pharmaceutical composition in unit dosage form which comprises about 100—400 milligrams of nicotinamide in admixture with an amount of an organic acid addition salt of m-AMSA sufficient to provide upon water reconstitution about 20—100 milligrams of m-AMSA.

29. A process according to anyone of claims 21—28, wherein the aqueous solution of step a) is filtered prior to lyophilization.

30. A process for preparing a stable, solid, water-soluble pharmaceutical composition comprising an organic acid addition salt of m-AMSA with a pharmaceutically acceptable organic acid, in admixture with nicotinamide and a pharmaceutically acceptable organic acid, the m-AMSA and nicotinamide being present in a ratio of about 2:40 to 1:1 which comprises

    A) mixing an organic acid salt of m-AMSA with nicotinamide and a pharmaceutically acceptable organic acid in the desired ratios; or
    B) the steps of
        a) forming an aqueous solution of an organic acid addition salt of m-AMSA, nicotinamide and a pharmaceutically acceptable organic acid in the desired ratios and
        b) lyophilizing the so-produced aqueous solution.

18

**0 042 553**

31. A process as claimed in claim 30, wherein the m-AMSA salt and organic acid are in a molar ratio of about 1:1.

32. A process as claimed in claim 30 or 31 wherein the m-AMSA salt is m-AMSA lactate, m-AMSA gluconate, m-AMSA citrate, m-AMSA levulinate, m-AMSA tartrate, m-AMSA pyroglutamate or m-AMSA glucoheptonate and the organic acid is tartaric acid, lactic acid, levulinic acid, citric acid, gluconic acid, pyroglutamic acid or glucoheptonic acid.

33. A process as claimed in claim 30 or 31 wherein the m-AMSA salt is m-AMSA lactate and the organic acid is lactic acid.

34. A process as claimed in claim 33, wherein the m-AMSA lactate is m-AMSA L(+)-monolactate hemiacetonate.

35. A process as claimed in claim 33, wherein the m-AMSA lactate is m-AMSA DL-monolactate acetonate having about 0.6—0.7 moles of acetone per mole of lactate.

36. A process as claimed in claim 33, wherein the m-AMSA lactate is m-AMSA D(−)-monolactate hemiacetonate.

37. A process as claimed in claim 30 or 31, wherein the m-AMSA salt is m-AMSA-gluconate and the organic acid is gluconic acid.

38. A process as claimed in anyone of claims 30 to 37 for preparing a pharmaceutical composition in unit dosage form comprising an amount of an organic acid addition salt of m-AMSA sufficient to provide upon water reconstitution about 20 to 100 milligrams of m-AMSA in admixture with about one molar equivalent of a pharmaceutically acceptable organic acid and 100 to 400 milligrams of nicotinamide.

39. A process according to claim 38 for preparing a pharmaceutical composition comprising an amount of m-AMSA lactate sufficient to provide upon water reconstitution about 50 milligrams of m-AMSA, one molar equivalent of lactic acid and 300 to 400 milligrams of nicotinamide.

40. A process according to anyone of claims 30—39 wherein the m-AMSA salt is m-AMSA lactate and the organic acid is D(−)-lactic acid, L(+)-lactic acid or a mixture of 50% D(−)- and 50% L(+)-lactic acid.

41. A process according to anyone of claims 30—40 wherein the aqueous solution of step a) is filtered prior to lyophilization.

**Patentansprüche für die Vertragsstaaten: BE, CH, DE, FR, GB, IT, LI, LU, NL, SE**

1. Stabile, feste, wasserlösliche pharmazeutische Zusammensetzung, die m - AMSA - [4' - (9 - Acridinylamino) - methansulfon - m - anisidid] in Mischung mit einer pharmazeutisch verträglichen organischen Säure und Nikotinamid enthält, wobei m-AMSA und Nikotinamid in einem Gewichtsverhältnis von m-AMSA: Nikotinamid von etwa 1:1 bis 1:20 vorhanden sind und die organische Säure und m-AMSA in einem Molverhältnis von organischer Säure: m-AMSA von etwa 1:1 bis 2:1 vorhanden sind.

2. Zusammensetzung nach Anspruch 1, worin die organische Säure Weinsäure, Milchsäure, Lävulinsäure, Zitronensäure, Gluconsäure, Pyroglutaminsäure oder Glucoheptonsäure ist.

3. Zusammensetzung nach Anspruch 2, worin die organische Säure Pyroglutaminsäure ist.

4. Zusammensetzung nach Anspruch 2, worin die organische Säure Milchsäure ist.

5. Zusammensetzung nach Anspruch 2, worin die organische Säure Gluconsäure ist.

6. Stabile, feste, wasserlösliche pharmazeutische Zusammensetzung, die ein organisches Säureadditionssalz von m-AMSA mit einer pharmazeutisch verträglichen organischen Säure in Mischung mit Nikotinamid enthält, wobei m-AMSA und Nikotinamid in einem Verhältnis von etwa 2:40 bis 1:1 vorhanden sind.

7. Zusammensetzung nach Anspruch 6, worin das m-AMSA-Salz m-AMSA-Lactat, m-AMSA-Gluconat, m-AMSA-Citrat, m-AMSA-Lävulinat, m-AMSA-Tartrat, m-AMSA-Pyroglutamat oder m-AMSA-Glucoheptonat ist.

8. Zusammensetzung nach Anspruch 6, worin das m-AMSA-Salz m-AMSA-Lactat ist.

9. Zusammensetzung nach Anspruch 8, worin das m-AMSA-Lactat-Salz m-AMSA-L(+)-Monolactat-hemiacetonat ist.

10. Zusammensetzung nach Anspruch 8, worin das m-AMSA-Lactat-Salz m-AMSA-D(−)-Monolactat-hemiacetonat ist.

11. Zusammensetzung nach Anspruch 8, worin das m-AMSA-Lactat m-AMSA-DL-Monolactat-acetonat mit etwa 0,6 bis 0,7 Mol Aceton pro Mol Lactat ist.

12. Zusammensetzung nach Anspruch 6, worin das m-AMSA-Salz m-AMSA-Gluconat ist.

13. Stabile, feste, wasserlösliche pharmazeutische Zusammensetzung, die ein organisches Säureadditionssalz von m-AMSA mit einer pharmazeutisch verträglichen organischen Säure in Mischung mit Nikotinamid und einer pharmazeutischen verträglichen organischen Säure enthält, wobei m-AMSA und Nikotinamid in einem Verhältnis von etwa 2:40 bis etwa 1:1 vorhanden sind.

14. Zusammensetzung nach Anspruch 13, worin das m-AMSA-Salz und die organische Säure in einem Molverhältnis von etwa 1:1 vorliegen.

15. Zusammensetzung nach Anspruch 13 oder 14, worin das m-AMSA-Salz, m-AMSA-Lactat, m-AMSA-Gluconat, m-AMSA-Citrat, m-AMSA-Lävulinat, m-AMSA-Tartrat, m-AMSA-Pyroglutamat oder m-AMSA-Glucoheptonat ist und die organische Säure Weinsäure, Milchsäure, Lävulinsäure, Zitronensäure, Gluconsäure, Pyroglutaminsäure oder Glucoheptonsäure ist.

19

16. Zusammensetzung nach Anspruch 13 oder 14, worin das m-AMSA-Salz m-AMSA-Lactat ist und die organische Säure Milchsäure ist.

17. Zusammensetzung nach Anspruch 16, worin das m-AMSA-Lactat m-AMSA-L(+)-Monolactat-hemiacetonat ist.

18. Zusammensetzung nach Anspruch 16, worin das m-AMSA-Lactat m-AMSA-DL-Monolactat-acetonat mit etwa 0,6 bis 0,7 Mol Aceton pro Mol Lactat ist.

19. Zusammensetzung nach Anspruch 16, worin das m-AMSA-Lactat m-AMSA-DL-Monolactat-hemiacetonat ist.

20. Zusammensetzung nach Anspruch 13 oder 14, worin m-AMSA-Salz m-AMSA-Gluconat ist und die organische Säure Gluconsäure ist.

21. Pharmazeutische Zusammensetzung nach Anspruch 4 in Form einer Dosiseinheit, die etwa 20 bis 100 mg m-AMSA in Mischung mit etwa 1 bis 2 Moläquivalenten Milchsäure und 100 bis 400 mg Nikotinamid enthält.

22. Zusammensetzung nach Anspruch 21, worin die Milchsäure L(+)-Milchsäure ist.

23. Zusammensetzung nach Anspruch 21, worin die Milchsäure DL-Milchsäure ist.

24. Zusammensetzung nach Anspruch 21, worin die Milchsäure D(−)-Milchsäure ist.

25. Zusammensetzung nach Anspruch 21, die etwa 20 bis 100 mg m-AMSA, 100 bis 400 mg Nikotinamid und 13 bis 26 mg Milchsäure enthält.

26. Zusammensetzung nach Anspruch 21, die etwa 50 mg m-AMSA, 300 bis 400 mg Nikotinamid und 26 mg Milchsäure enthält.

27. Pharmazeutische Zusammensetzung nach Anspruch 2 in Form einer Dosiseinheit, die etwa 10 bis 30 mg m-AMSA in Mischung mit etwa 1 bis 2 Moläquivalenten Zitronensäure und 100 bis 400 mg Nikotinamid enthält.

28. Zusammensetzung nach Anspruch 27, die etwa 20 bis 30 mg m-AMSA, 1 bis 2 Moläquivalente Zitronensäure und 300 bis 400 mg Nikotinamid enthält.

29. Zusammensetzung nach Anspruch 27, die etwa 20 mg m-AMSA, 10 mg Zitronensäure und 300 bis 400 mg Nikotinamid enthält.

30. Pharmazeutische Zusammensetzung nach Anspruch 2 in Form einer Dosiseinheit, die etwa 20 bis 100 mg m-AMSA in Mischung mit etwa 1 bis 2 Moläquivalenten Gluconsäure oder Gluconolacton und 100 bis 400 mg Nikotinamid enthält.

31. Zusammensetzung nach Anspruch 30, die etwa 75 mg m-AMSA, 37 bis 74 mg Gluconsäure oder Gluconolacton und 300 bis 400 mg Mikotinamid enthält.

32. Pharmazeutische Zusammensetzung nach einem der Ansprüche 6 bis 12 in Einheitsdosisform, die 100 bis 400 mg Nikotinamid in Mischung mit einer solchen Menge eines organischen Säureadditionssalzes von m-AMSA enthält, daß nach Rekonstitution mit Wasser 20 bis 100 mg m-AMSA bereitgestellt werden.

33. Pharmazeutische Zusammensetzung nach einem der Ansprüche 13 bis 20 in Form einer Dosiseinheit, die eine ausreichende Menge eines organischen Säureadditionssalzes von m-AMSA, um nach Rekonstitution mit Wasser etwa 20 bis 100 mg m-AMSA bereitzustellen, in Mischung mit etwa einem Moläquivalent einer pharmazeutisch verträglichen organischen Säure und 100 bis 400 mg Nikotinamid enthält.

34. Zusammensetzung nach Anspruch 33, die eine ausreichende Menge an m-AMSA-Lactat, um nach Rekonstitution mit Wasser etwa 50 mg m-AMSA bereitzustellen, ein Moläquivalent Milchsäure und 300 bis 400 mg Nikotinamid enthält.

35. Pharmazeutische Zusammensetzung nach Anspruch 2 in Einheitsdosisform, die 50 mg m-AMSA, 17 bis 35 mg L-Pyroglutaminsäure und 350 mg Nikotinamid enthält.

36. Verfahren zur Herstellung einer Zusammensetzung nach irgend einem der Ansprüche 1 bis 5, 21 bis 31 und 35 wobei man

A) m-AMSA mit einer pharmazeutisch verträglichen organischen Säure und Nikotinamid in den gewünschten Verhältnissen mischt oder

B) folgende Stufen durchführt:
a) Herstellen einer wäßrigen Lösung aus einem organischen Säureadditionssalz von m-AMSA, einer pharmazeutisch verträglichen organischen Säure und Nikotinamid in den gewünschten Verhältnissen und
b) Lyophilisieren der so hergestellten wäßrigen Lösung.

37. Verfahren nach Anspruch 36, wobei man als organische Säure in Verfahren A D(−)-Milchsäure, L(+)-Milchsäure, eine Mischung von 50% D-(−)- und 50% L(+)-Milchsäure oder Gluconolacton einsetzt.

38. Verfahren nach Anspruch 36, wobei man die wäßrige Lösung der Stufe a) vor der Lyophilisierung filtriert.

39. Verfahren nach Anspruch 36 oder 38, wobei man als organische Säure in Stufe a) Milchsäure, Gluconsäure oder Gluconolacton einsetzt.

40. Verfahren zur Herstellung einer Zusammensetzung nach einem der Ansprüche 6 bis 12 und 32 wobei man

A) ein organisches Säureadditionssalz von m-AMSA mit Nikotinamid in dem gewünschten Verhältnis vermischt oder

B) folgende Stufen durchführt,
 a) Herstellen einer wäßrigen Lösung aus einem organischen Säureadditionssalz von m-AMSA und Nikotinamid in dem gewünschten Verhältnis, und
 b) Lyophilisieren der so hergestellten Lösung.

41. Verfahren nach Anspruch 40, wobei man die wäßrige Lösung in Stufe a) vor der Lyophilisierung filtriert.

42. Verfahren zur Herstellung einer Zusammensetzung nach irgend einem der Ansprüche 13 bis 30, 32 und 34, wobei man

A) ein organisches Säureadditionssalz von m-AMSA mit Nikotinamid und einer pharmazeutisch verträglichen organischen Säure in den gewünschten Verhältnissen mischt oder

B) folgende Stufen durchführt:
 a) Herstellen einer wäßrigen Lösung aus einem organischen Säureadditionssalz von m-AMSA, Nikotinamid und einer pharmazeutisch verträglichen organischen Säure in den gewünschten Verhältnissen und
 b) Lyophilisieren der so hergestellten wäßrigen Lösung.

43. Verfahren nach Anspruch 42, wobei das m-AMSA-Salz m-AMSA-Lactat ist und die organische Säure D-(−)-Milchsäure, L(+)-Milchsäure oder eine Mischung von 50% D(−)- und 50% L(+)-Milchsäure ist.

44. Verfahren nach Anspruch 42 oder 43, worin man die wäßrige Lösung von Stufe a) vor der Lyophilisierung filtriert.

**Patentansprüche für den Vertragsstaat AT**

1. Verfahren zur Herstellung einer stabilen, festen, wasserlöslichen pharmazeutischen Zusammensetzung, die m-AMSA[4' - (9 - Acridinylamino) - methansulfon - m - anisidid] in Mischung mit einer pharmazeutisch verträglichen organischen Säure und Nikotinamid enthält, wobei m-AMSA und Nikotinamid in einem Gewichtsverhältnis von m-AMSA: Nikotinamid von etwa 1:1 bis 1:20 vorhanden sind und die organische Säure und m-AMSA in einem Molverhältnis von organischer Säure: m-AMSA von etwa 1:1 bis 2:1 vorhanden sind, wobein man

A) m-AMSA mit einer pharmazeutisch verträglichen organischen Säure und Nikotinamid in den gewünschten Verhältnissen mischt oder

B) folgende Stufen durchführt:
 a) Herstellen einer wäßrigen Lösung aus einem organischen Säureadditionssalz von m-AMSA, einer pharmazeutisch verträglichen organischen Säure und Nikotinamid in den gewünschten Verhältnissen; und
 b) Lyophilisieren der so hergestellten wäßrigen Lösung.

2. Verfahren nach Anspruch 1, worin die eingesetzte organische Säure Weinsäure, Milchsäure, Lävulinsäure, Zitronensäure, Gluconsäure, Pyroglutaminsäure oder Glucoheptonsäure ist.

3. Verfahren nach Anspruch 2, worin die organische Säure Pyroglutaminsäure ist.

4. Verfahren nach Anspruch 2, worin die organische Säure Milchsäure ist.

5. Verfahren nach Anspruch 2, worin die organische Säure Gluconsäure ist.

6. Verfahren nach Anspruch 4 zur Herstellung einer pharmazeutischen Zusammensetzung in Form einer Dosiseinheit die etwa 20 bis 100 Milligramm m-AMSA in Mischung mit etwa 1 bis 2 Moläquivalenten Milchsäure und 100 bis 400 Milligramm Nikotinamid enthält.

7. Verfahren nach Anspruch 6, worin die Milchsäure L(+)-Milchsäure ist.

8. Verfahren nach Anspruch 6, worin die Milchsäure DL-Milchsäure ist.

9. Verfahren nach Anspruch 6, worin die Milchsäure D(−)-Milchsäure ist.

10. Verfahren nach Anspruch 6 zur Herstellung einer pharmazeutischen Zusammensetzung, die etwa 20 bis 100 mg m-AMSA, 100—400 Milligramm Nikotinamid und 13—26 Milligramm Milchsäure enthält.

11. Verfahren nach Anspruch 6 zur Herstellung einer pharmazeutischen Zusammensetzung, die etwa 50 Milligramm m-AMSA, 300—400 Milligramm Nikotinamid und 26 Milligramm Milchsäure enthält.

12. Verfahren nach Anspruch 2 zur Herstellung einer pharmazeutischen Zusammensetzung in Form einer Dosiseinheit form, die etwa 10 bis 30 Milligramm m-AMSA in Mischung mit etwa 1 bis 2 Moläquivalenten Zitronensäure und 100 bis 400 Milligramm Nikotinamid enthält.

13. Verfahren nach Anspruch 12 zur Herstellung einer pharmazeutischen Zusammensetzung, die etwa 20 bis 30 Milligramm m-AMSA, 1 bis 2 Moläquivalente Zitronensäure und 300 bis 400 Milligramm Nikotinamid enthält.

14. Verfahren nach Anspruch 12 zur Herstellung einer pharmazeutischen Zusammensetzung, die etwa 20 bis 100 Milligramm m-AMSA, 10 mg Zitronensäure und 300 bis 400 Milligramm Nikotinamid enthält.

**0 042 553**

15. Verfahren nach Anspruch 2 zur Herstellung einer pharmazeutischen Zusammensetzung in Form einer Dosiseinheit, die etwa 20 bis 100 Milligramm m-AMSA in Mischung mit etwa 1 bis 2 Moläquivalenten Gluconsäure oder Gluconolactan und 100 bis 400 Milligramm Nikotinamid enthält.

16. Verfahren nach Anspruch 15 zur Herstellung einer pharmazeutischen Zusammensetzung, die etwa 75 Milligramm m-AMSA, 37—74 Milligramm Gluconsäure oder Gluconolacton und 300—400 Milligramm Nikotinamid enthält.

17. Verfahren nach Anspruch 2 zur Herstellung einer pharmazeutischen Zusammensetzung in Form einer Dosiseinheit, die 50 mg m-AMSA, 17—35 mg L-Pyroglutaminsäure und 350 mg Nikotinamid enthält.

18. Verfahren nach einem der vorhergehenden Ansprüche, worin die organische Säure in Stufe A D(−)-Milchsäure, L(+)-Milchsäure, eine Mischung von 50% D-(−)-und 50% L(+)-Milchsäure oder Gluconolacton ist.

19. Verfahren nach einem der vorhergehenden Ansprüche, worin die wäßrige Lösung in Stufe a) vor der Lyophilisierung filtriert wird.

20. Verfahren nach den Ansprüchen 1—17 oder Anspruch 19, worin die organische Säure in Stufe a) Milchsäure, Gluconsäure oder Gluconolacton ist.

21. Verfahren zur Herstellung einer stabilen, festen, wasserlöslichen pharmazeutischen Zusammensetzung, die ein organisches Säureadditionssalz von m-AMSA mit einer pharmazeutisch verträglichen organischen Säure in Mischung mit Nikotinamid enthält, wobei m-AMSA und Nikotinamid in einem Verhältnis von 2:40 bis 1:1 vorhanden sind, wobei man

A) ein organisches Säureadditionssalz von m-AMSA mit Nikotinamid in dem gewünschten Verhältnis mischt oder

B) folgende Stufen durchführt,
a) Herstellen einer wäßrigen Lösung aus einem organischen Säureadditionssalzes von m-AMSA und Nikotinamid in dem gewünschten Verhältnis; und
b) Lyophilisieren der so hergestellten Lösung.

22. Verfahren nach Anspruch 21, worin das m-AMSA Salz m-AMSA Lactat, m-AMSA Gluconat, m-AMSA Citrat, m-AMSA Levulinat, m-AMSA Tartrat, m-AMSA Pyroglutamat oder m-AMSA Glucoheptonat ist.

23. Verfahren nach Anspruch 21, worin das m-AMSA Salz m-AMSA Lactat ist.

24. Verfahren nach Anspruch 23, worin das m-AMSA Lactat m-AMSA L(+)-Monolactathemiacetonat ist.

25. Verfahren nach Anspruch 23, worin das m-AMSA Lactat-salz m-AMSA D(−)-Monolactathemiacetonat ist.

26. Verfahren nach Anspruch 23, worin das m-AMSA-Lactat m-AMSA DL-Monolactatacetonat mit etwa 0,6—0,7 Mol Aceton pro Mol Lactat ist.

27. Verfahren nach Anspruch 21, worin das m-AMSA-Salz m-AMSA Gluconat ist.

28. Verfahren nach irgendeinem der Ansprüche 21—27 zur Herstellung einer pharmazeutischen Zusammensetzung in Form einer Dosiseinheit, die etwa 100—400 Milligramm Nikotinamid in Mischung mit einer solchen Menge eines organischen Säureadditionssalzes von m-AMSA enthält, daß nach Rekonstitution mit Wasser etwa 20—100 Milligramm m-AMSA bereitgestellt werden.

29. Verfahren nach irgendeinem der vorhergehenden Ansprüche 21—28, worin die wäßrige Lösung der Stufe a) vor der Lyophilisierung filtriert wird.

30. Verfahren zur Herstellung einer stabilen, festen, wasserlöslichen pharmazeutischen Zusammensetzung, die ein organisches Säureadditionssalz von m-AMSA mit einer pharmazeutisch verträglichen organischen Säure in Mischung mit Nikotinamid und einer pharmazeutisch verträglichen organischen Säure enthält, wobei m-AMSA und Nikotinamid in einem Verhältnis von etwa 2:40 bis 1:1 vorhanden sind, wobei man

A) ein organisches Säuresalz von m-AMSA mit Nikotinamid und einer pharmazeutisch verträglichen organischen Säure in den gewünschten Verhältnissen mischt; oder

B) folgende Stufen durchführt,
a) Herstellen einer wäßrigen Lösung aus einem organischen Säureadditionssalz von m-AMSA, Nikotinamid und einer pharmazeutisch verträglichen organischen Säure in den gewünschten Verhältnissen und
b) Lyophilisieren der so hergestellten wäßrigen Lösung.

31. Verfahran nach Anspruch 30, worin das m-AMSA Salz und die organische Säure in einem Molverhältnis von etwa 1:1 vorhanden sind.

32. Verfahren nach Anspruch 30 oder 31, worin das m-AMSA Salz m-AMSA Lactat, m-AMSA Gluconat, m-AMSA Citrat, m-AMSA Lävulinat, m-AMSA Tartrat, m-AMSA Pyroglutamat oder m-AMSA Glucoheptonat ist und die organische Säure Weinsäure, Milchsäure, Lavulinsäure, Zitronensäure, Gluconsäure, Pyroglutaminsäure oder Glucoheptonsäure ist.

22

33. Verfahren nach Anspruch 30 oder 31, worin das m-AMSA Salz m-AMSA Lactat und die organische Säure Milchsäure ist.

34. Verfahren nach Anspruch 33, worin das m-AMSA Lactat m-AMSA L(+)-Monolactathemiacetonat ist.

35. Verfahren nach Anspruch 33, worin das m-AMSA Lactat m-AMSA DL-Monolactatacetonat mit 0,6—0,7 Mol Aceton pro Mol Lactat ist.

36. Verfahren nach Anspruch 33, worin das m-AMSA Lactat m-AMSA D(−)-Monolactathemiacetonat ist.

37. Verfahren nach Anspruch 30 oder 31, worin das m-AMSA Salz m-AMSA-Gluconat und die organische Säure Gluconsäure ist.

38. Verfahren nach irgendeinem der Ansprüche 30 bis 37 zur Herstellung einer pharmzeutischen Zusammmensetzung in Form einer Dosiseinhiet, die eine ausreichende Menge eines organischen Säureadditionssalzes von m-AMSA, um nach Rekonstitution mit Wasser etwa 20 bis 100 Milligramm m-AMSA bereitzustellen, im Mischung mit etwa einem Moläquivalent einer pharmazeutischen verträglichen organischen Säure und 100 bis 400 Milligramm Nikotinamid enthält.

39. Verfahren nach Anspruch 38 zur Herstellung einer pharmazeutischen Zusammensetzung, die eine ausreichende Menge m-AMSA Lactat, um nach Rekonstitution mit Wasser etwa 50 Milligramm m-AMSA bereitzustellen, ein Moläquivalent Milchsäure und 300 bis 400 Milligramm Nikotinamid enthält.

40. Verfahren nach irgendeinem der Ansprüche 30—39, worin das m-AMSA Salz m-AMSA Lactat ist und die organische Säure D(−)-Milchsäure, L(+)-Milchsäure oder eine Mischung von 50% D(−)- und 50% L(+)-Milchsäure ist.

41. Verfahren nach irgendeinem der Ansprüche 30—40, worin die wäßrige Lösung der Stufe a) vor der Lyophilisierung filtriert wird.

**Revendications pour les Etats Contractants: BE, CH, DE, FR, GB, IT, LI, LU, NL, SE**

1. Composition pharmaceutique stable, solide et soluble dans l'eau comprenant du m-AMSA [4' - (9 - acridinylamino) - méthanesulfon - m - anisidide] en mélange avec un acide organique pharmaceutiquement acceptable et du nicotinamide, le m-AMSA et le nicotinamide étant présents dans un rapport pondéral m-AMSA: nicotinamide d'environ 1:1 à 1:20, l'acide organique et le m-AMSA étant présents dans un rapport molaire acide organique: m-AMSA d'environ 1:1 à 2:1.

2. Composition suivant la revendication 1, dans laquelle l'acide organique est l'acide tartrique, l'acide lactique, l'acide lévulinique, l'acide citrique, l'acide gluconique, l'acide pyroglutamique ou l'acide glucoheptonique.

3. Composition suivant la revendication 2, dans laquelle l'acide organique est l'acide pyroglutamique.

4. Composition suivant la revendication 2, dans laquelle l'acide organique est l'acide lactique.

5. Composition suivant la revendication 2, dans laquelle l'acide organique est l'acide gluconique.

6. Composition pharmaceutique stable, solide et soluble dans l'eau comprenant un sel d'addition d'acide organique ou m-AMSA avec un acide organique pharmaceutiquement acceptable, en mélange avec du nicotinamide, le m-AMSA et le nicotinamide étant présents dans un rapport d'environ 2:40 à 1:1.

7. Composition suivant la revendication 6, dans laquelle le sel de m-AMSA est le lactate de m-AMSA, le gluconate de m-AMSA, le citrate de m-AMSA, le lévulinate de m-AMSA, le tartrate de m-AMSA, le pyroglutamate de m-AMSA ou le glucoheptonate de m-AMSA.

8. Composition suivant la revendication 6, dans laquelle le sel de m-AMSA est le lactate de m-AMSA.

9. Composition suivant la revendication 8, dans laquelle le lactate de m-AMSA est le L(+)-monolactate héniacétonate de m-AMSA.

10. Composition suivant la revendication 8, dans laquelle le lactate de m-AMSA est le D(−)-monolactate hémiacétonate de m-AMSA.

11. Composition suivant la revendication 8, dans laquelle le lactate de m-AMSA est le DL-monolactate acétonate de m-AMSA comprenant environ 0,6—0,7 mole d'acétone par mole de lactate.

12. Composition suivant la revendication 6, dans laquelle le sel de m-AMSA est le gluconate de m-AMSA.

13. Composition pharmaceutique stable, solide et soluble dans l'equ comprenant un sel d'addition d'acide organique du m-AMSA avec un acide organique pharmaceutiquement acceptable, en mélange avec du nicotinamide et un acide organique pharmaceutiquement acceptable, le m-AMSA et le nicotinamide étant présents dans un rapport d'environ 2:40 à 1:1.

14. Composition suivant la revendication 13, dans laquelle le sel de m-AMSA et l'acide organique sont présents dans un rapport molaire d'environ 1:1.

15. Composition suivant la revendication 13 ou 14, dans laquelle le sel de m-AMSA est le lactate de m-AMSA, le gluconate de m-AMSA, le citrate de m-AMSA, le lévulinate de m-AMSA, le tartrate de m-AMSA, le pyroglutamate de m-AMSA ou le glucoheptonate de m-AMSA et l'acide organique est l'acide tartrique, l'acide lactique, l'acide lévulinique, l'acide citrique, l'acide gluconique, l'acide pyroglutamique ou l'acide glucoheptonique.

16. Composition suivant la revendication 13 ou 14, dans laquelle le sel de m-AMSA et le lactate de m-AMSA et l'acide organique est l'acide l'actique.

**0 042 553**

17. Composition suivant la revendication 16, dans laquelle le lactate de m-AMSA est le L(+)-monolactate hémiacétonate de m-AMSA.

18. Composition suivant la revendication 16, dans laquelle le lactate de m-AMSA est le DL-monolactate acétonate de m-AMSA comprenant environ 0,6—0,7 mole d'acétone par mole de lactate.

19. Composition suivant la revendication 16, dans laquelle le lactate de m-AMSA est le D(−)-monolactate hémiacétonate de m-AMSA.

20. Composition suivant la revendication 13 ou 14, dans laquelle le sel de m-AMSA est le gluconate de m-AMSA et l'acide organique est l'acide gluconique.

21. Composition pharmaceutique suivant la revendication 4 sous forme dosée unitaire qui comprend environ 20 à 100 mg de m-AMSA en mélange avec environ 1 à 2 équivalents molaires d'acide lactique et 100 à 400 mg de nicotinamide.

22. Composition suivant la revendication 21, dans laquelle l'acide lactique est l'acide L(+)-lactique.

23. Composition suivant la revendication 21, dans laquelle l'acide lactique est l'acide DL-lactique.

24. Composition suivant la revendication 21, dans laquelle l'acide lactique est l'acide D(−)-lactique.

25. Composition suivant la revendication 21, qui comprend environ 20 à 100 mg de m-AMSA, 100—400 mg de nicotinamide et 13—26 mg d'acide lactique.

26. Composition suivant la revendication 21, qui comprend environ 50 mg de m-AMSA, 300—400 mg de nicotinamide et 26 mg d'acide lactique.

27. Composition pharmaceutique suivant la revendication 2 sous forme dosée unitaire qui comprend environ 10 à 30 mg de m-AMSA en mélange avec environ 1 à 2 équivalents molaires d'acide citrique et 100 à 400 mg de nicotinamide.

28. Composition suivant la revendication 27, qui comprend environ 20 à 30 mg de m-AMSA, 1 à 2 équivalents molaires d'acide citrique et 300 à 400 mg de nicotinamide.

29. Composition suivant la revendication 27, qui comprend environ 20 mg de m-AMSA, 10 mg d'acide citrique et 300 à 400 mg de nicotinamide.

30. Composition pharmaceutique suivant la revendication 2 sous forme dosée unitaire qui comprend environ 20 à 100 mg de m-AMSA en mélange avec environ 1 à 2 équivalents molaires d'acide gluconique ou de gluconolactone et 100 à 400 mg de nicotinamide.

31. Composition suivant la revendication 30, qui comprend environ 75 mg de m-AMSA, 37—74 mg d'acide gluconique ou de gluconolactone et 300—400 mg de nicotinamide.

32. Composition pharmaceutique suivant l'une quelconque des revendications 6—12 sous forme dosée unitaire qui comprend environ 100 à 400 mg de nicotinamide en mélange avec une quantité d'un sel d'addition d'acide organique du m-AMSA suffisante pour donner par dilution avec de l'eau environ 20 à 100 mg de m-AMSA.

33. Composition pharmaceutique suivant l'une quelconque des revendications 13 à 20 sous forme dosée unitaire qui comprend une quantité d'un sel d'addition d'acide organique du m-AMSA suffisante pour apporter par dilution avec de l'eau environ 20 à 100 mg de m-AMSA en mélange avec environ un équivalent molaire d'un acide organique pharmaceutiquement acceptable et 100 à 400 mg de nicotinamide.

34. Composition suivant la revendication 33, qui comprend une quantité de lactate de m-AMSA suffisante pour apporter par dilution avec de l'eau environ 50 mg de m-AMSA, un équivalent molaire d'acide lactique et 300 à 400 mg de nicotinamide.

35. Composition pharmaceutique suivant la revendication 2 sous forme dosée unitaire qui comprend 50 mg de m-AMSA, 17—35 mg d'acide L-pyroglutamique et 350 mg de nicotinamide.

36. Procédé de préparation d'une composition suivant l'une quelconque des revendications 1—5, 21—31 et 35, qui comprend

    A) le mélange du m-AMSA avec un acide organique pharmaceutiquement acceptable et du nicotinamide dans les rapports souhaités ou

    B) les stades de
      a) former une solution aqueuse d'un sel d'addition d'acide organique du m-AMSA, d'un acide organique pharmaceutiquement acceptable et de nicotinamide dans les rapports souhaités et
      b) lyophiliser la solution aqueuse ainsi obtenue.

37. Procédé suivant la revendication 36, dans lequel l'acide organique pour le procédé A est l'acide D(−)-lactique, l'acide L(+)-lactique, un mélange de 50% d'acide D-(−)-lactique et de 50% d'acide L(+)-lactique ou la gluconolactone.

38. Procédé suivant la revendication 36, dans lequel la solution aqueuse du stade a) est filtrée avant la lyophilisation.

39. Procédé suivant la revendication 36 ou la revendication 38, dans lequel l'acide organique au stade a) est l'acide lactique, l'acide gluconique ou la gluconolactone.

40. Procédé de préparation d'une composition suivant l'une quelconque des revendications 6 à 12 et 32, qui comprend

24

A) le mélange d'un sel d'addition d'acide organique du m-AMSA avec du nicotinamide dans le rapport souhaité ou

B) les stades de

a) former une solution aqueuse d'un sel d'addition d'acide organique du m-AMSA et de nicotinamide dans le rapport souhaité et

b) lyophiliser la solution ainsi obtenue.

41. Procédé suivant la revendication 40, dans lequel la solution aqueuse du stade a) est filtrée avant la lyophilisation.

42. Procédé de préparation d'une composition suivant l'une quelconque des revendications 13—20, 33 et 34, qui comprend

A) le mélange d'un sel d'addition d'acide organique du m-AMSA avec du nicotinamide et un acide organique pharmaceutiquement acceptable dans les rapports souhaités ou

B) les stades de

a) former une solution aqueuse d'un sel d'addition d'acide organique du m-AMSA, du nicotinamide et d'un acide organique pharmaceutiquement acceptable dans les rapports souhaités et

b) lyophiliser la solution aqueuse ainsi obtenue.

43. Procédé suivant la revendication 42, dans lequel le sel de m-AMSA est le lactate de m-AMSA et l'acide organique est l'acide D(−)-lactique, l'acide L(+)-lactique ou un mélange de 50% d'acide D(−)-lactique et de 50% d'acide L(+)lactique.

44. Procédé suivant la revendication 42 ou 43, dans lequel la solution aqueuse du stade (a) est filtrée avant la lyophilisation.

**Revendications pour l'Etat Contractant AT**

1. Procédé de préparation d'une composition pharmaceutique stable, solide et soluble dans l'eau comprenant du m-AMSA [4' - (9 - acridinylamino) - méthanesulfon - m - anisidide] en mélange avec un acide organique pharmaceutiquement acceptable et du nicotinamide, le m-AMSA et le nicotinamide étant présents dans un rapport pondéral m-AMSA: nicotinamide d'environ 1:1 à 1:20, l'acide organique et le m-AMSA étant présents dans un rapport molaire acide organique: m-AMSA d'environ 1:1 à 2:1, qui comprend

A) le mélange du m-AMSA avec un acide organique pharmaceutiquement acceptable et du nicotinamide dans les rapports souhaités ou

B) les stades de

a) former une solution aqueuse d'un sel d'addition d'acide organique du m-AMSA, d'un acide organique pharmaceutiquement acceptable et de nicotinamide dans les rapports souhaités et

b) lyophiliser la solution aqueuse ainsi obtenue.

2. Procédé suivant la revendication 1, dans lequelle l'acide organique est l'acide tartrique, l'acide lactique, l'acide lévulinique, l'acide citrique, l'acide gluconique, l'acide pyroglutamique ou l'acide glucoheptonique.

3. Procédé suivant la revendication 2, dans lequelle l'acide organique est l'acide pyroglutamique.

4. Procédé suivant la revendication 2, dans lequelle l'acide organique est l'acide lactique.

5. Procédé suivant la revendication 2, dans lequelle l'acide organique est l'acide gluconique.

6. Procédé suivant la revendication 4 pour la préparation d'une composition pharmaceutique sous forme dosée unitaire qui comprend environ 20 à 100 mg de m-AMSA en mélange avec environ 1 à 2 équivalents molaires d'acide lactique et 100 à 400 mg de nicotinamide.

7. Procédé suivant la revendication 6, dans lequelle l'acide lactique est l'acide L(+)-lactique.

8. Procédé suivant la revendication 6, dans lequelle l'acide lactique est l'acide DL-lactique.

9. Procédé suivant la revendication 6, dans lequelle l'acide lactique est l'acide D(−)-lactique.

10. Procédé suivant la revendication 6, pour la préparation d'une composition pharmaceutique qui comprend environ 20 à 100 mg de m-AMSA, 100—400 mg de nicotinamide et 13—26 mg d'acide lactique.

11. Procédé suivant la revendication 6 pour la préparation d'une composition pharmaceutique, qui comprend environ 50 mg de m-AMSA, 300—400 mg de nicotinamide et 26 mg d'acide lactique.

12. Procédé suivant la revendication 2 pour la préparation d'une composition sous forme dosée unitaire qui comprend environ 10 à 30 mg de m-AMSA en mélange avec environ 1 à 2 équivalents molaires d'acide citrique et 100 à 400 mg de nicotinamide.

13. Procédé suivant la revendication 12 pour la préparation d'une composition pharmaceutique qui comprend environ 20 à 30 mg de m-AMSA, 1 à 2 équivalents molaires d'acide citrique et 300 à 400 mg de nicotinamide.

14. Procédé suivant la revendication 12 pour la préparation d'une composition pharmaceutique qui comprend environ 20 mg de m-AMSA, 10 mg d'acide citrique et 300 à 400 mg de nicotinamide.

15. Procédé suivant la revendication 2 pour la préparation d'une composition pharmaceutique sous forme dosée unitaire qui comprend environ 20 à 100 mg de m-AMSA en mélange avec environ 1 à 2 équivalents molaires d'acide gluconique ou de gluconolactone et 100 à 400 mg de nicotinamide.

16. Procédé suivant la revendication 15 pour la préparation d'une composition pharmaceutique, qui comprend environ 75 mg de m-AMSA, 37—74 mg d'acide gluconique ou de gluconolactone et 300—400 mg de nicotinamide.

17. Procédé suivant la revendication 2 pour la préparation d'une composition pharmaceutique sous forme dosée unitaire qui comprend 50 mg de m-AMSA, 17—35 mg d'acide L-pyroglutamique et 350 mg de nicotinamide.

18. Procédé suivant une des revendications précédentes, dans lequel l'acide organique pour le procédé A est l'acide D(−)-lactique, l'acide L(+)-lactique, un mélange de 50% d'acide D-(−)-lactique et de 50% d'acide L(+)-lactique ou la gluconolactone.

19. Procédé suivant une des revendications précédentes, dans lequel la solution aqueuse du stade a) est filtrée avant la lyophilisation.

20. Procédé suivant les revendications 1—17 ou la revendication 19, dans lequel l'acide organique au stade a) est l'acide lactique, l'acide gluconique ou la gluconolactone.

21. Procédé de préparation d'une composition pharmaceutique stable, solide et soluble dans l'eau comprenant un sel d'addition d'acide organique du m-AMSA avec un acide organique pharmaceutiquement acceptable, en mélange avec du nicotinamide, le m-AMSA et le nicotinamide étant présents dans un rapport d'environ 2:40 à 1:1, qui comprend

    A) le mélange d'un sel d'addition d'acide organique du m-AMSA avec du nicotinamide dans le rapport souhaité ou

    B) les stades de
        a) former une solution aqueuse d'un sel d'addition d'acide organique du m-AMSA et de nicotinamide dans le rapport souhaité et
        b) lyophiliser la solution ainsi obtenue.

22. Procédé suivant la revendication 21, dans lequelle le sel de m-AMSA est le lactate de m-AMSA, le gluconate de m-AMSA, le citrate de m-AMSA, le lévulinate de m-AMSA, le tartrate de m-AMSA, le pyroglutamate de m-AMSA ou le glucoheptonate de m-AMSA.

23. Procédé suivant la revendication 21, dans lequelle le sel de m-AMSA est le lactate de m-AMSA.

24. Procédé suivant la revendication 23, dans lequelle le lactate de m-AMSA est le L(+)-monolactate hémiacétonate de m-AMSA.

25. Procédé suivant la revendication 23, dans lequelle le lactate de m-AMSA est le D(−)-monolactate hémiacétonate de m-AMSA.

26. Procédé suivant la revendication 23, dans lequelle le lactate de m-AMSA est le DL-monolactate acétonate de m-AMSA comprenant environ 0,6—0,7 mole d'acétone par mole le lactate.

27. Composition suivant la revendication 21, dans laquelle le sel de m-AMSA est le gluconate de m-AMSA.

28. Procédé suivant l'une quelconque des revendications 21—27 pour la préparation d'une composition pharmaceutique forme dosée unitaire qui comprend environ 100 à 400 mg de nicotinamide en mélange avec une quantité d'un sel d'addition d'acide organique du m-AMSA suffisante pour donner par dilution avec de l'eau environ 20 à 100 mg de m-AMSA.

29. Procédé suivant l'une quelconque des revendications 21—28 dans lequel la solution aqueuse du stade a) est filtrée avant la lyophilisation.

30. Procédé de préparation d'une composition pharmaceutique stable, solide et soluble dans l'eau comprenant un sel d'addition d'acide organique du m-AMSA avec un acide organique pharmaceutiquement acceptable, en mélange avec du nicotinamide et un acide organique pharmaceutiquement acceptable, le m-AMSA et le nicotinamide étant présents dans un rapport d'environ 2:40 à 1:1, qui comprend

    A) le mélange d'un sel d'addition d'acide organique du m-AMSA avec du nicotinamide et un acide organique pharmaceutiquement acceptable dans les rapports souhaités ou

    B) les stades de
        a) former une solution aqueuse d'un sel d'addition d'acide organique du m-AMSA, du nicotinamide et d'un acide organique pharmaceutiquement acceptable dans les rapports souhaités et
        b) lyophiliser la solution aqueuse ainsi obtenue.

31. Procédé suivant la revendication 30, dans lequelle le sel de m-AMSA et l'acide organique sont présents dans un rapport molaire d'environ 1:1.

32. Procédé suivant la revendication 30 ou 31, dans lequelle le sel de m-AMSA est le lactate de m-AMSA, le gluconate de m-AMSA, le citrate de m-AMSA, le lévulinate de m-AMSA, le tartrate de m-AMSA, le pyroglutamate de m-AMSA ou le glucoheptonate de m-AMSA et l'acide organique est l'acide

**0 042 553**

tartrique, l'acide lactique, l'acide lévulinique, l'acide citrique, l'acide gluconique, l'acide pyroglutamique ou l'acide glucoheptonique.

33. Procédé suivant la revendication 30 ou 31, dans lequelle le sel de m-AMSA est le lactate de m-AMSA et l'acide organique est l'acide lactique.

34. Procédé suivant la revendication 33, dans lequelle le lactate de m-AMSA est le L(+)-monolactate hémiacétonate de m-AMSA.

35. Procédé suivant la revendication 33, dans lequelle le lactate de m-AMSA est le DL-monolactate acétonate de m-AMSA comprenant environ 0,6—0,7 mole d'acétone par mole de lactate.

36. Procédé suivant la revendication 33, dans lequelle le lactate de m-AMSA est le D(−)-monolactate hémiacétonate de m-AMSA.

37. Procédé suivant la revendication 30 ou 31, dans lequelle le sel de m-AMSA est le gluconate de m-AMSA et l'acide organique est l'acide gluconique.

38. Procédé suivant l'une quelconque des revendications 30—37 pour la préparation d'une composition pharmaceutique sous forme dosée unitaire qui comprend une quantité d'un sel d'addition d'acide organique du m-AMSA suffisante pour apporter par dilution avec de l'eau environ 20 à 100 mg de m-AMSA en mélange avec environ un équivalent molaire d'un acide organique pharmaceutiquement acceptable et 100 à 400 mg de nicotinamide.

39. Procédé suivant la revendication 38, pour la préparation d'une composition pharmaceutique qui comprend une quantité de lactate de m-AMSA suffisante pour apporter par dilution avec de l'eau environ 50 mg de m-AMSA, un équivalent molaire d'acide lactique et 300 à 400 mg de nicotinamide.

40. Procédé suivant l'une quelconque des revendications 30—39, dans lequel le sel de m-AMSA est le lactate de m-AMSA et l'acide organique est l'acide D(−)-lactique, l'acide L(+)-lactique ou un mélange de 50% d'acide D(−)-lactique et de 50% d'acide L(+)lactique.

41. Procédé suivant l'une quelconque des revendications 30—40, dans lequel la solution aqueuse du stade a) est filtrée avant la lyophilisation.

27